# EUROPEAN PATENT SPECIFICATION

(11) **EP 2 967 547 B1**
(45) Date of publication and mention of the grant of the patent: **24.10.2018**
(21) Application number: 14763595.7
(22) Date of filing: 14.03.2014
(51) Int. Cl.: A61B 17/04, A61B 17/06, A61B 17/11, A61B 90/30, A61B 17/00, A61B 17/30

(54) **SUTURE DEVICE**
NAHTVORRICHTUNG
DISPOSITIF DE SUTURE

(30) Priority: 15.03.2013 US 201361799877 P
(43) Date of publication of application: 20.01.2016
(73) Proprietor: TC1 LLC, St. Paul, MN 55117 (US)
(72) Inventor: HOARAU, Carine, Pleasant Hill, California 94523 (US); REICHENBACH, Steven H., Pleasanton, California 94588 (US); HSU, George, San Ramon, California 94583 (US); JACOBS, Robert A., Grass Valley, California 95949 (US)
(74) Representative: ZBM Patents - Zea, Barlocci & Markvardsen
(86) International application number: PCT/US2014/029703
(87) International publication number: WO 2014/145053

(56) References cited:
- EP-A1- 2 277 457
- WO-A2-2005/065412
- US-A1- 2004 049 194
- US-A1- 2005 251 209
- US-A1- 2007 112 384
- US-A1- 2010 275 432
- US-A1- 2011 288 563
- US-A1- 2011 301 619
- US-A1- 2012 101 525
- US-A1- 2012 165 931
- US-A1- 2012 221 021

## Description

### FIELD

This invention relates, in general, to a suture device.

### BACKGROUND

Mechanical circulatory support has increasingly become a standard of care for advanced heart failure. An example of a circulatory support system is a left ventricle assist device (LVAD) for pumping blood from the heart to the ascending aorta. The LVAD is typically implanted at the apex of the left ventricle of the patient's heart. This is often accomplished by the use of an attachment ring, also referred to as a cuff, made of silicon and felt. In a conventional implantation procedure, a surgeon sutures the attachment ring to the base of the left ventricle with at least 12 pledgeted horizontal mattress 2-0 braided sutures almost full thickness through the myocardium. Corresponding sutures are then applied to the felt cuff. The sutures must then be separated and tied tight to gather the myocardium around the felt cuff. This is a time consuming process that is also open to the possibility of human error and inconsistency by the surgeon.

Document US 2010/0275432 shows a device for sealing an opening in a vessel wall and/or tissue tract including an anchor, a filament, a plug, and a knot. The anchor is configured to be disposed adjacent to an interior surface of the vessel wall adjacent the opening in the vessel wall. The filament has a distal end coupled to the anchor and the plug is disposed about at least a portion of the filament.

Document WO 2005/065142 shows devices and method for attachment of an implanted device within a patient's digestive tract for treatment of obesity having surgical fasteners. Such fasteners have a T-shape and are attached to the suture. The T-shaped fasteners provide a secure attachment to the gastrointestinal tissue without causing excessive pressure within the tissue and are adapted for convenient and reliable deployment

What is needed is a system that overcomes these and other problems. What is needed is a system that can automate at least part of the process without risking malpositioning of the cuff and sutures.

### SUMMARY OF THE DISCLOSURE

The present invention is directed to a suture device comprising a suture core having a core inner lumen, a suture crimp positioned at a proximal end of the suture core and fastened to a suture within the core inner lumen, and an anchoring sleeve positioned at a distal end of the suture core. The anchoring sleeve is compressible in an axial direction, is secured to a distal-most end of the suture core, and extends in a proximal direction. The anchoring sleeve is adapted to collapse and anchor the distal end of the suture core against a tissue wall.

### BRIEF DESCRIPTION OF THE DRAWINGS

The novel features of the invention are set forth with particularity in the claims that follow. A better understanding of the features and advantages of the present invention will be obtained by reference to the following detailed description that sets forth illustrative embodiments, in which the principles of the invention are utilized, and the accompanying drawings of which:
FIG. 1 is a sectional view of an exemplary suture device in accordance with the invention.
FIG. 2 is a sectional view of the suture penetrating tissue.
FIG. 3 is a sectional view of the suture being retracted from the tissue and compressing the anchoring sleeve.
FIG. 4 is an isometric view of an exemplary attachment ring for attachment to a left ventricle of a heart.
FIG. 5 is a sectional view of the attachment ring.
FIG. 6 is an isometric view of an exemplary pneumatic suture delivery tool.
FIG. 7 is a sectional view of the pneumatic suture delivery tool.
FIG. 8 is a sectional view of the pneumatic suture delivery tool crimping mechanism.
FIG. 9 is an isometric view of the lower piston with needles and sutures.
FIG. 10 is an isometric view showing the attachment ring insertion into the tool.
FIG. 11 is an isometric view of the attachment ring inside the tool.
FIG. 12 is an isometric view of the tool positioned on the left ventricle.
FIG. 13 is a sectional view showing the left ventricle conforming to the tissue vacuum cup.
FIG. 14 is a sectional view of the needle insertion process.
FIG. 15 is an isometric view of the suture insertion process.
FIG. 16 is an isometric view of the needle extraction and cinching process using the suture delivery tool.
FIGS. 17A and 17B are plan views of exemplary needles.
FIG. 18 is a cross-section view showing a distal segment of an exemplary suture core.
FIG. 19 is a cross-section view the attachment ring of FIG. 5 secured to tissue by suture devices of FIG. 1.
FIG. 20 is a disassembled, side view of an exemplary coupling system for securing an anastomotic prosthesis to biological tissue, showing a delivery tool, an attachment ring, and an engagement device.
FIG. 21 is a disassembled, perspective view of the coupling system of FIG. 20.
FIGS. 22-25 are perspective, top, bottom, and side views of the engagement device of FIG. 20.
FIGS. 26 and 27 are cross-section views of the engagement device of FIG. 20, showing the engagement device in perspective and from the side.
FIG. 28 is a plan view of a clip which can be carried within and deployed out of the delivery tool of FIG. 20.
FIG. 29 is a perspective view of the engagement device of FIG. 20, showing the engagement device detached from the delivery tool having a connection device.
FIG. 30 is a perspective view of the engagement device of FIG. 20, showing the engagement device secured to the delivery tool by the connection device.
FIG. 31 is another coupling system for securing for securing an anastomotic prosthesis to biological tissue, showing the delivery tool of FIG. 20 with another engagement device.
FIG. 32 is a detailed cross-section view of a forward portion of the coupling system of FIG. 31, showing the delivery tool and engagement device in perspective.
FIG. 33 is a side, cross-section view of the engagement device and delivery tool of FIG. 30.
FIGS. 34 and 35 are perspective and cross-section views of an exemplary ring-shaped contact member for use with the devices of FIGS. 20 and 31, showing a frustoconical contact surface.
FIG. 36 is a perspective view of an exemplary ring-shaped contact member for use with the devices of FIG. 20 and 31, showing accessory attachment points.
FIGS. 37A-37E are perspective views of an exemplary applicator tool for anchoring an attachment ring to biological tissue using securement clips, the applicator tool shown fully assembled in FIG. 37A, disassembled in FIGS. 37B-37D, and close-up in FIG. 37E.

### DETAILED DESCRIPTION

Reference will now be made in detail to the preferred embodiments of the invention, examples of which are illustrated in the accompanying drawings. While the invention will be described in conjunction with the preferred embodiments, it will be understood that they are not intended to limit the invention to those embodiments.

For convenience in explanation and accurate definition in the appended claims, the terms "up" or "upper", "down" or "lower", "inside" and "outside" are used to describe features of the present invention with reference to the positions of such features as displayed in the figures.

In many respects the modifications of the various figures resemble those of preceding modifications and the same reference numerals followed by subscripts "a", "b", "c", and "d" designate corresponding parts.

The invention relates to an improved system for performing anastomosis, and in various respects, connecting a prosthesis to tissue. In various embodiments, the system is configured to connect a vascular prosthesis to an organ such as the apex of the left ventricle of a patient's heart. In various embodiments, the system is configured to connect the prosthesis to a body lumen such as an artery or vein. The system can be configured for various applications throughout the body including end-to-end attachment.

Various aspects of the system are similar to those described in International Pub. Nos. WO1995004503A1 to Domaas et al. and WO2001041623 to Keren et al.; U.S. Pub. Nos. 2013/0282026 to Hoarau et al., 2012/0221021 to Hoarau et al., 2010/0069932 to Bilotti et al., 2004/0260318 to Gravett et al., and 2004/0068217 to Brenzel et al.; and U.S. Patent Nos. 5,695,504 to Bolduc et al., 6,361,559 to Fleischman et al., 6,638,237 to Guiles et al., 7,309,341 to Ortiz et al., and 7,682,368 to Bombard et al.

Turning now to the drawings, the exemplary automatic anastomosis system includes a suture device 28, an attachment member, and a pneumatic suture delivery tool 29. The exemplary suture device is a self-anchoring suture device. The exemplary suture delivery tool incorporates a plurality of NiTinol tubular needles. The exemplary attachment member is a ventricular cuff for a left ventricular assist device (LVAD), right ventricular assist device (RVAD), or Bi-ventricular assist device (BiVAD). The attachment member is in the shape of ring and made from silicone and felt with integrated metal suture crimps. Although shown and described as a ring for attachment to a ventricular apex, one will appreciate from the description herein that various other structures may be used in place of the attachment member. In various embodiments, the delivery tool is configured to deliver the suture devices without an attachment ring, for example, in a tissue-to-tissue application.

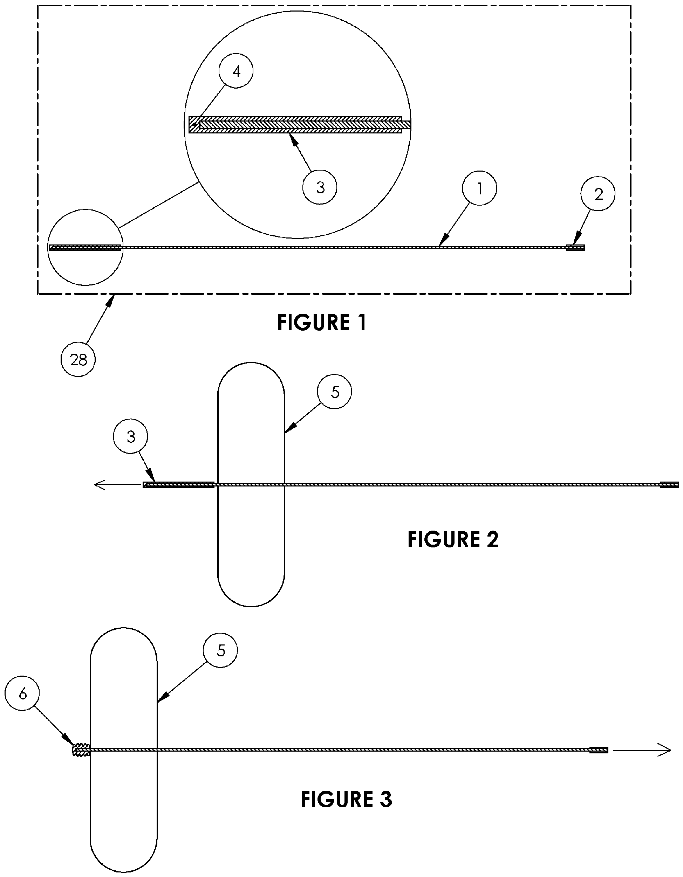

Sectional views of the suture device 28 can be seen in Figures 1, 2, and 3. The suture includes three components: a suture core 1, a cinching crimp 2, and an anchoring sleeve 3. The exemplary suture core is 2-0 braided polyester. Other suture materials may be used. The exemplary cinching crimp 2 is made of a rigid metal (e.g. titanium). Although referred to and described as a cinching crimp, one will appreciate from the description herein that other shapes and configurations may be used. For example, other types of fastening devices may be used, including non-mechanical fasteners. The exemplary an anchoring sleeve 3 is adapted to be collapsible in an axial direction. The anchoring sleeve may be configured to collapse when subjected to a compressive force above a predefined threshold. The exemplary anchoring sleeve is made from 3-0 braided polyester. Although referred to and described as a sleeve, one will appreciate from the description herein that other shapes and configurations may be used.

In one embodiment a proximal end of the suture core 1 is threaded through the center of the cinching crimp 2 and is mechanically crimped in place. The distal end of the suture core 1 is then threaded through the center of the anchoring sleeve 3 and fastened to the distal tip 4 of the suture core 1. In an exemplary embodiment, the sleeve and core are thermally fused using a laser.

As shown in Figure 2, the exemplary suture device penetrates tissue 5 in a first axial direction, represented by the direction of the arrow, until the full length of the anchoring sleeve 3 has cleared the tissue 5. The suture is then retracted in a second direction, represented by the direction of the arrow in Figure 3, resulting in a compressed anchoring sleeve 6. In the exemplary embodiment, the second direction is substantially opposite the first direction. The compression of the sleeve restricts the suture from being drawn back through the tissue 5 as shown in Figure 3. The anchoring sleeve thus prevents retraction of the suture after insertion through the tissue. In other words, the anchoring sleeve member is adapted to allow the suture to be moved in only one direction through the tissue. In this manner, the suture is anchored to the tissue.

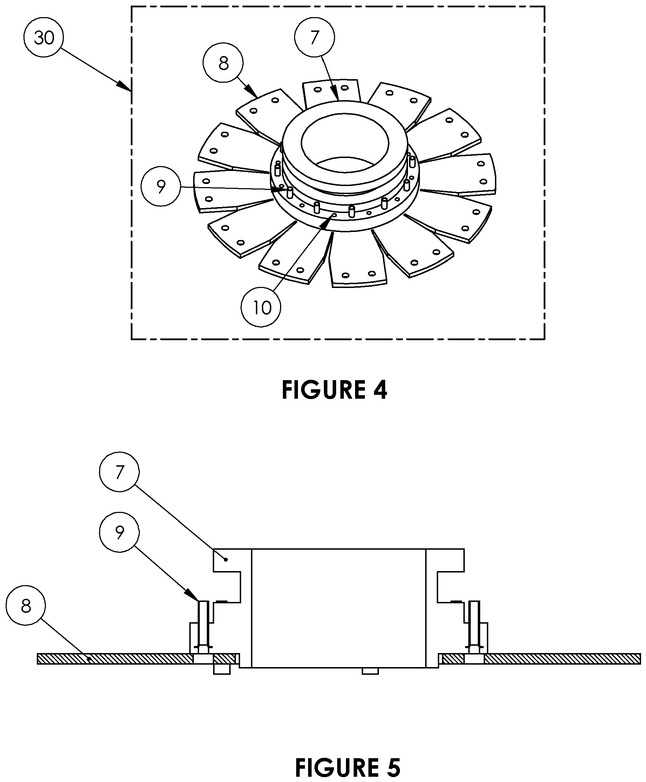

Figures 4 and 5 show the components that comprise the exemplary attachment ring 30. In one embodiment, the cannula seal 7 is molded from silicone and is attached to a Dacron or PTFE felt ring 8 by means of sutures through suture holes 10. Retaining crimps 9 are provided for the self-anchoring sutures. The exemplary retaining crimps are made from flared titanium tubes and molded into the silicone.

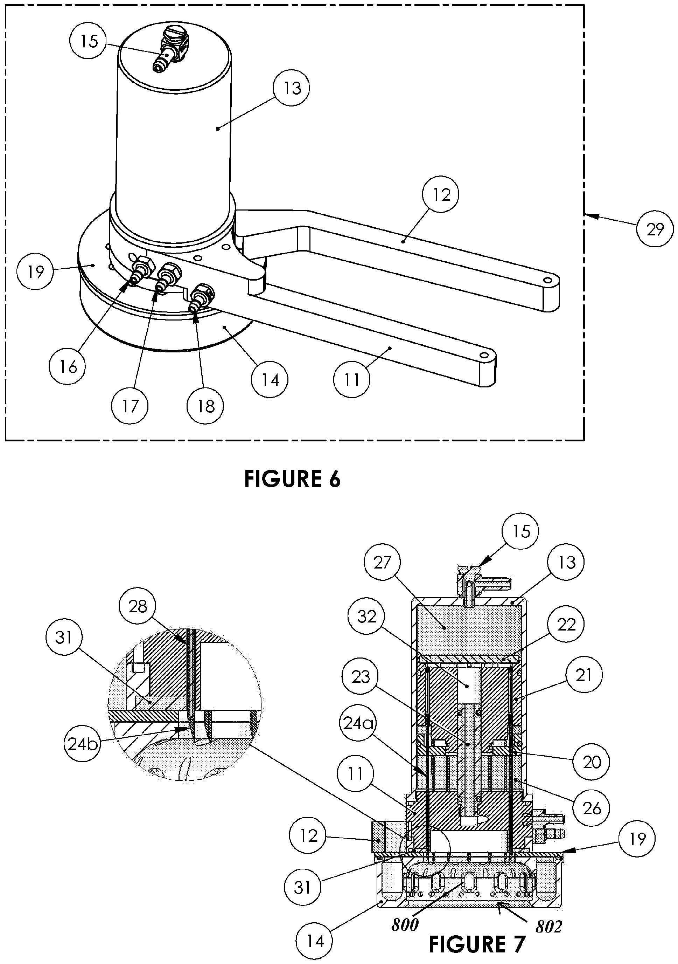

Figures 6, 7, and 8 show the components that comprise the exemplary pneumatic suture delivery tool 29. In the preferred embodiment, the main structure consists of a stationary handle 11, a cam ring handle 12, a cylinder 13, a crimp retainer 19, and a tissue vacuum cup 14. Inside the cylinder 13 is a lower piston 20, an upper piston 21, a piston cap 22, and a piston stem 23. Bonded to the lower piston 20 are twelve NiTinol tubular needles 24 (shown in retracted state 24a and extended state 24b).

The upper piston chamber 27 is connected by a pneumatic channel to a needle insertion port 15 while a lower piston chamber 26 is connected by a pneumatic channel to a needle retraction port 17. The piston stem 23 that provides the air to drive the sutures 28 through the tubular NiTinol needles 24a is connected by a pneumatic channel to the suture insertion port 16. The tissue vacuum cup 14 is connected by a pneumatic channel to an external vacuum port 18. Held captive within the stationary handle 11 by the cam ring handle 12 and the crimp retainer 19 are twelve crimp dies 31.

One will appreciate from the description herein that the suture delivery tool may be modified within the scope of the invention. For example, the tool may be actuated by means other than pneumatics such as an electric drive. The tool may include any number of dies and crimps, and means other than crimping may be employed.

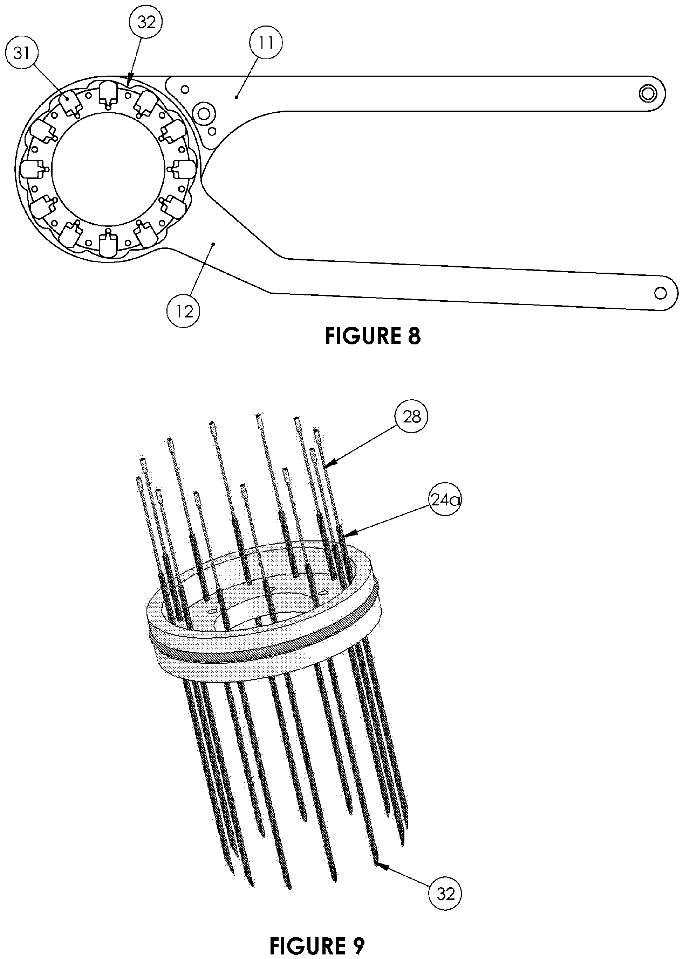

Figures 9 through 16 illustrate the exemplary suture device, attachment ring, and delivery device in use, and in particular, the operation of the exemplary delivery tool for attaching the ring to the myocardium at the apex of a left ventricle. As shown in Figure 9, the twelve tubular Nitinol needles 24a are drawn into a retracted state. Twelve sutures 28 are then inserted into the NiTinol needles 24a such that the tip of the anchoring sleeve 3 is just inside the tip of the NiTinol needle 32.

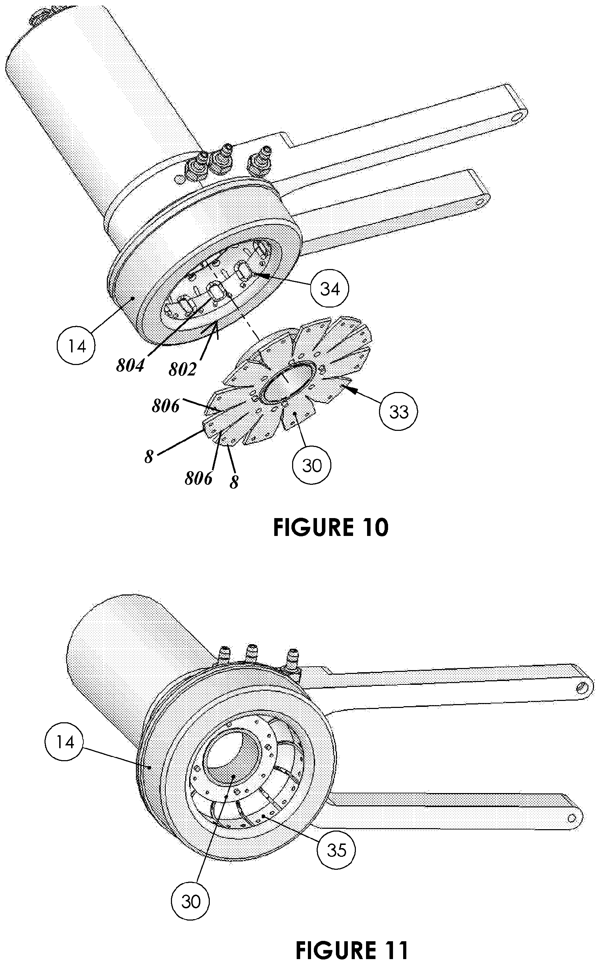

The attachment ring 30 is then inserted into the tissue vacuum cup 14 such that the felt retaining holes 33 in the PTFE felt ring 8 align with the locating nubs 34 (shown in Figure 10). This assures that the flaps 35 of the PTFE felt ring 8 will stay positioned in the tissue vacuum cup 14 when the tubular NiTinol needles 24 penetrate the felt. Once inserted, the felt flaps 35 of the attachment ring 30 should conform to the shape of the inner cavity of the tissue vacuum cup 14 as shown in Figure 11.

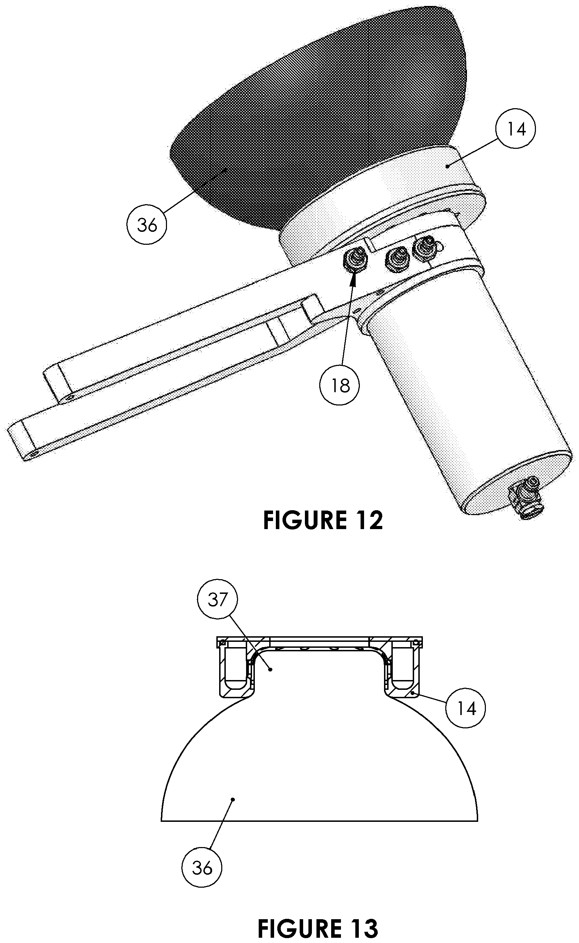

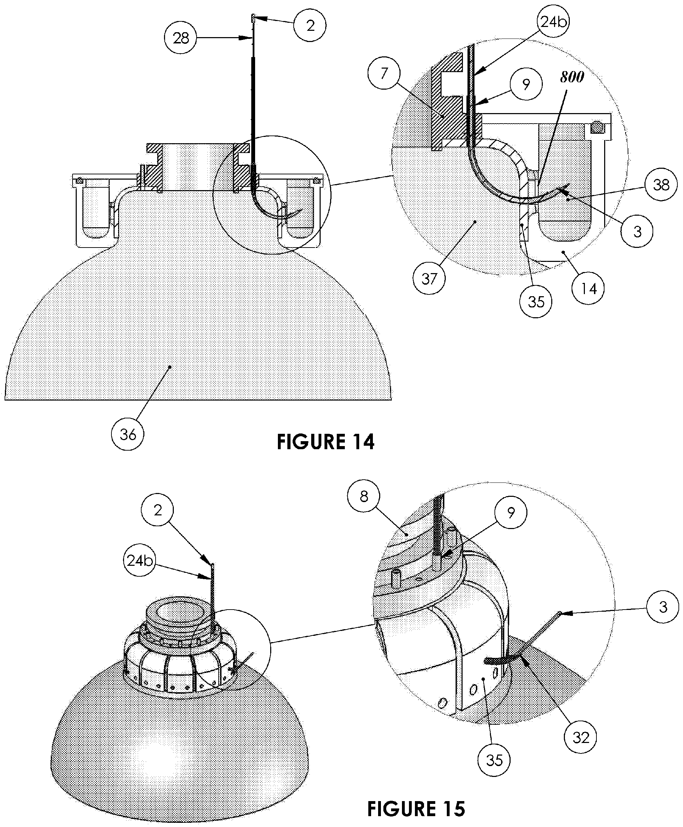

With the attachment ring 30 inserted, the tissue vacuum cup 14 is positioned over the end of the left ventricle 36 where the attachment ring is to be attached (shown in Figure 12). A vacuum source is then applied to the external vacuum port 18 which is in fluid communication with vacuum chamber 38 (FIG. 14). The vacuum draws air out of vacuum chamber 38 which is connected by apertures 800 to central chamber 802 (FIG. 10) of vacuum cup 14. The vacuum produces suction in central chamber 802 via apertures 800 and vacuum chamber 38. Suction passes through slits 806 between flaps 8 of attachment ring 30 so that the suction is applied to the tissue below attachment ring 30. The vacuum is slowly increased, such as through the use of a regulator or valve mechanism connected to the vacuum source, until the myocardium 37 of the left ventricle 36 is drawn by the suction into central chamber 802 of tissue vacuum cup 14, which causes the tissue to conform to the inner shape of the cup as shown in Figure 13. Specifically, tissue conforms to the inner shape of central chamber 802. This configuration assures that the tubular NiTinol needles 24b will have consistent penetration into the myocardium when deployed.

The clinician examines the tissue position and determines if it is in a desired location. The clinician may evaluate the tissue to ensure that it is completely conformed to the inner wall of the cup so the tissue is assured to be in alignment with the delivery path of the needles. Once satisfied with the positioning of the attachment ring 30 on the left ventricle 36, a predefined air pressure is applied via a pneumatic switch to the needle insertion port 15 inducing a pressure differential between upper piston chamber 27 and lower piston chamber 26. This drives the piston assembly downward. The tubular NiTinol needles 24b bonded to the lower piston 20 are thereby driven through the retaining crimps 9 of the attachment ring 30, into the myocardium 37 of the left ventricle 36, through the felt flaps 35, through apertures 800, and then exit into the inner vacuum chamber 38 of the tissue vacuum cup 14.

With the tubular NiTinol needles 24b deployed, a predefined air pressure is applied via a pneumatic switch to the suture insertion port 16. Air flows from the port 16 through the center of the piston stem 23 and into a manifold between the piston cap 22 and the upper piston 21 where it is distributed and directed into the twelve tubular NiTinol needles. As the air flows through the needles, it creates a friction boundary layer along the surface of the sutures 28 within the needles which propels the sutures toward the distal end of the tubular NiTinol needles 24b. The suture devices 28 come to rest when the cinching crimp 2 at the proximal end of the suture core 1 is stopped by the proximal end of the tubular NiTinol needle 24b. In an exemplary embodiment, suture devices 28 are of such a length that they come to rest only after the full length of the anchoring sleeve 3 has cleared the tip of the tubular NiTinol needle 32.

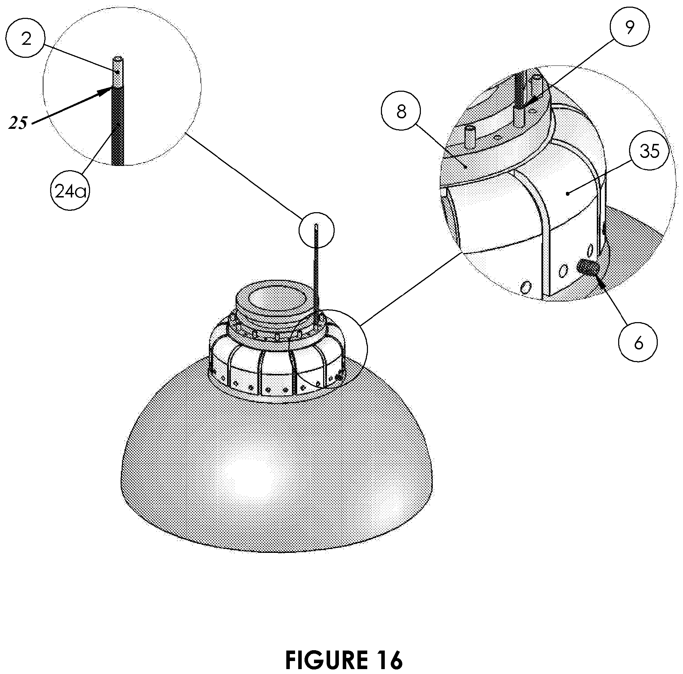

Next, air pressure at the suture insertion port 16 is removed by reversing the pneumatic switch to vent the upper piston chamber 27 to atmosphere through the needle insertion port 15. Air pressure at the needle retraction port 17 is slowly increased by means of a manually controlled regulator, thus increasing the pressure in the lower piston chamber 26. The increasing pressure in the lower piston chamber slowly drives the tubular NiTinol needles 24b up by means of the bond between the needles and the lower piston 20. As the proximal end 25 of each needle retracts, it bears against the cinching crimp 2 of each suture device and compresses the suture sleeves 6 against the felt flaps 35 (shown in Figure 16). This cinching process pulls the myocardium 37 of the left ventricle 36 tight against the PTFE felt ring 8. The exact tightness of this cinching process is advantageously determined by the pressure induced by the regulator at the needle retraction port 17.

After the correct cinching force has been achieved, the cam ring handle 12 is squeezed by hand towards the stationary handle 11. The rotational movement of the cam ring handle 12 translates into linear motion of the twelve crimp dies 31 by means of twelve cams 32 milled into the cam ring handle (shown in Figure 8). The crimp dies 31 bear down on the retaining crimps 9 of the attachment ring 30 thereby crimping the sutures 28 within. The 12 sutures are now held at the right tension securing the attachment ring 30 to the left ventricle 36.

Upon completion of the cinching process described above, attachment ring 30 is firmly secured to myocardium 37 of the left ventricle 36. Suction in central chamber 802 is discontinued to allow attachment ring 30 to separate from vacuum cup 14. Vacuum cup 14 can be lifted away from myocardium 37, which leaves attachment ring 30 attached to myocardium 37. Next, with delivery tool 29 removed from the heart, a cannulation procedure can be performed through attachment ring 30 to provide a fluid connection from the chamber of left ventricle 36 to a ventricular assist device.

A cannulation procedure may include placing a cutting instrument through the center of attachment ring 30 in order to make a hole through myocardium 37, followed by removal of the cutting instrument, followed by insertion of a cannula through the center of attachment ring 30 and into the chamber of the left ventricle 36, and then locking the cannula to the attachment ring 30. The cannula may already be connected to a ventricular assist device when it is inserted in the heart chamber, or the cannula may be subsequently connected to a ventricular assist device after the cannula has been inserted into the heart chamber.

Cannula seal 7 (FIG. 5) is configured to ensure blood flow through the central fluid passageway of the cannula and to prevent leakage of blood between the outer surface of the cannula and the interior of attachment ring 30. Cannula seal 7 can be an elastomeric O-ring or annular wiper seal that is configured to conform to the outer surface of the cannula.

To facilitate cannulation while the heart is beating, a valvular structure can be attached to attachment ring 30 before a hole is made through myocardium 37. Thereafter, the cannulation procedure described above can be performed by passing the cutting instrument through the valvular structure to make the hole through myocardium 37. When the cutting instrument is withdrawn, the valvular structure blocks blood flow and prevents exsanguination. Next, the cannula can be inserted through the valvular structure and attachment ring 30. After the cannula is locked onto attachment ring 30, valvular structure may be disassembled so that it can be removed from attachment ring 30 and the cannula. The valvular structure can include a housing that can be separated into multiple pieces, and further include flexible valve members in the housing. The housing and flexible valve members and be similar to those described in U.S. Application Publication No. 2011/0118766 A1.

Further information regarding the configuration of the delivery tool and its use are described in U.S. Pub. Nos. 2013/0282026 and 2012/0221021, both to Hoarau et al. Both U.S. Pub. Nos. 2013/0282026 and 2012/0221021 disclose delivery tools similar to delivery tool 29 in many respects except that the tool in those publications is configured to deploy solid needles whereas delivery tool 29 is configured to deploy needles 24 each having a needle inner lumen containing suture device 28.

In some embodiments, each needle 24 has a predefined shape in which it is curved when in a natural, unconstrained state similar to the clips described in Pub. Nos. 2013/0282026 and 2012/0221021. The axial length of needle 24 forms an arc. Suture device 28 may have slots, similar to those of the applicator tool in Pub. No. 2012/0221021, which carry and constrain needles 24 to be straight. When needles 24 are pushed out of the slots, the tip of needles 24 follow curved trajectories due to the tendency of needles 24 to return to their predefined curved shape.

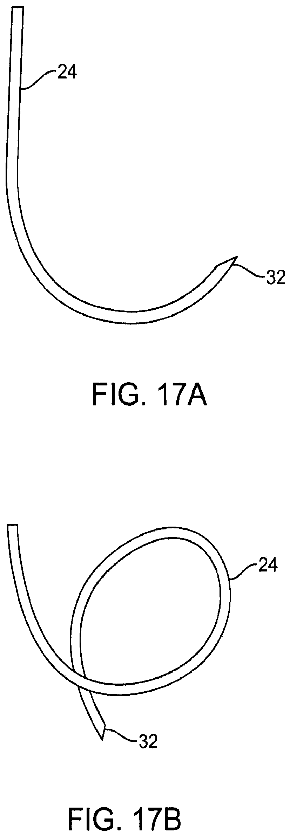

In some embodiments, as shown in FIG. 17A and 17B, needle 24 can have a predefined substantially arcuate shape. Needle 24 is arcuate in that its axial length forms an arc or loop. In the embodiment shown in FIG. 17A, the distal segment near tip 32 is arcuate. In another embodiment shown in FIG. 17B, needle 24 forms a loop in which one segment of needle 24 crosses over another segment of needle 24. In both FIGS. 17A and 17B, needle 24 is in its natural, unconstrained state. The arcuate shape of needle 24 allows tip 32 to travel along a curved path as shown in FIG. 14. When needle 24 is loaded into delivery tool 29, the needle 24 is elastically deformed and constrained in a straight configuration as shown in FIG. 9.

As discussed above, needle 24 can be made of Nitinol, which is a nickel titanium alloy that exhibits both shape memory and superelasticity. Shape memory allows needle 24 to undergo deformation at one temperature, and subsequently return to its original, undeformed shape when needle 24 is brought to a temperature above the transformation temperature of the alloy. Superelasticity allows the needle 24 to exhibit elasticity which is many times that of ordinary metal (for example, ordinary stainless steel) when needle 24 is within a temperature range above its transition temperature. Other alloys, such as copper-based alloys known in the art, may be used to make needle 24 in order to provide shape memory and superelasticity.

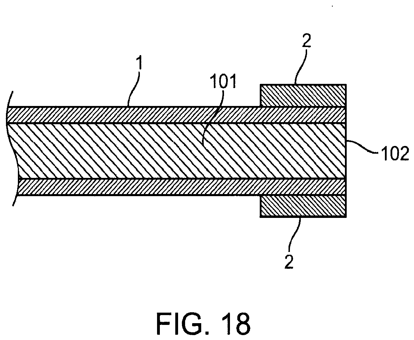

As shown in FIG. 18, suture core 1 includes a core inner lumen 101. FIG. 18 shows the proximal segment of suture device 28 of FIG. 9. Crimp 2 is adapted to fasten suture 102 within core inner lumen 101. Suture 102 is positioned within core inner lumen 101. Crimp 2 is in the form of an annular band which is configured to be compressed. When compressed, crimp 2 holds its compressed shape and thereby fastens suture 102 within core inner lumen 101.

As discussed above, a cinching process is performed by providing air pressure at needle retraction port 17, which results in retraction of proximal end 25 of needle 24 in a direction away from myocardium 37. The air pressure at needle retraction port 17 causes the needle 24 to be driven in a distal direction into cylinder 13 of delivery tool 29. Cinching can also be performed using pressure applied by hand through the use of other delivery tool designs (see descriptions under the heading Second Embodiment and Third Embodiment below).

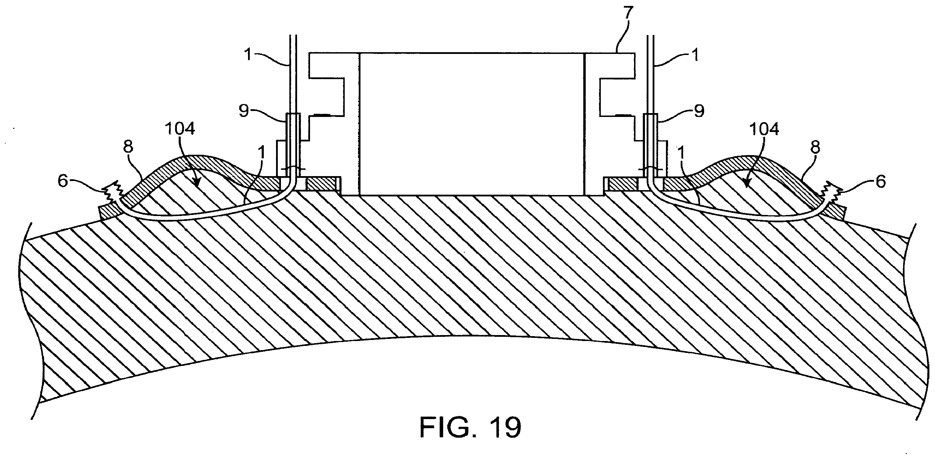

As shown in FIG. 19, after the cinching process is complete, thickened portion 104 of tissue is secured against flap 8 of attachment ring 30. FIG. 19 shows attachment ring 30 secured to the tissue after vacuum pressure has been discontinued and after the attachment ring 30 and the tissue have been released from vacuum cup 14 of delivery tool 29.

In some embodiments, the thickening of the tissue is uniform around flap 8. In some embodiments, the thickening of the tissue is non-uniform around flap 8. In some embodiments, thickening is uniform in thickness around flap 28. Thickened portion 104 is squeezed between curved segments of flap 8 so that a pressure is applied to thickened portion 104. The air pressure at needle retraction port 17 can be controlled so that driving of needle 24 in a distal direction can be performed to achieve a desired pressure on the tissue. Thickened portion 104 is under stress, and the amount of thickening is selected to achieve a desired pre-stressing of the tissue.

### SECOND EMBODIMENT

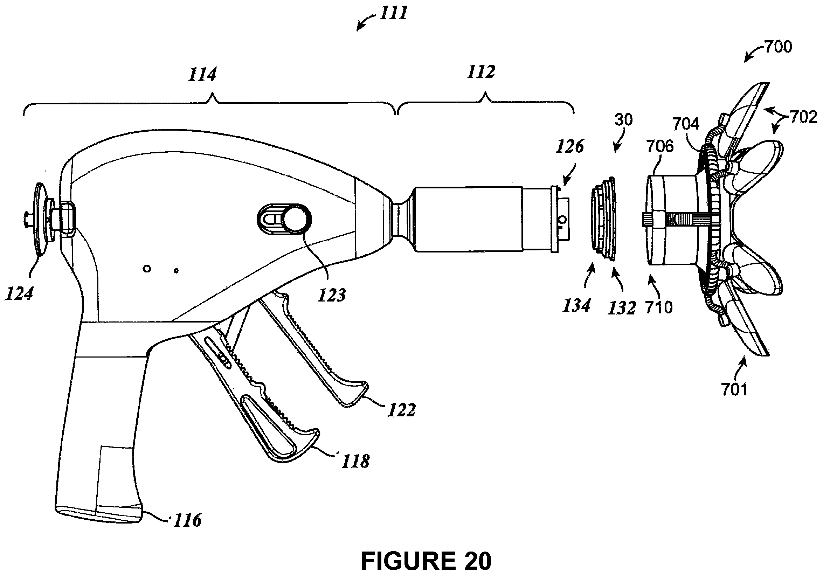

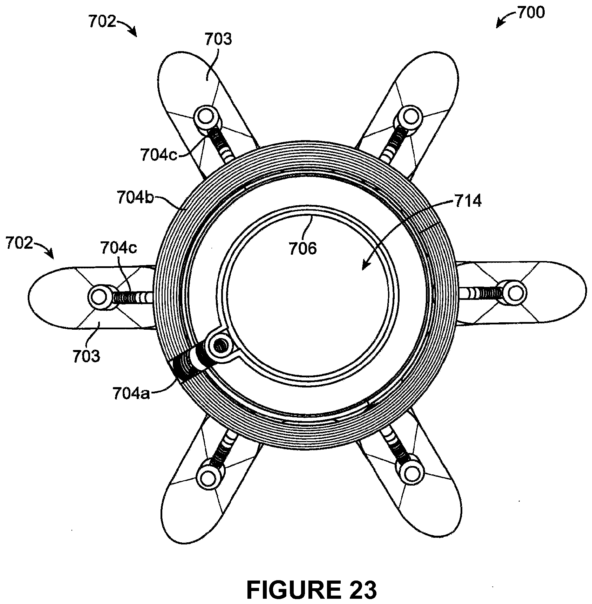

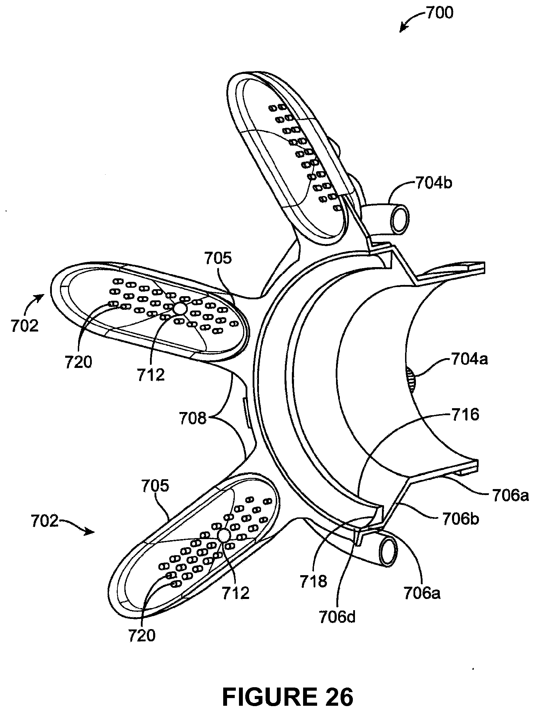

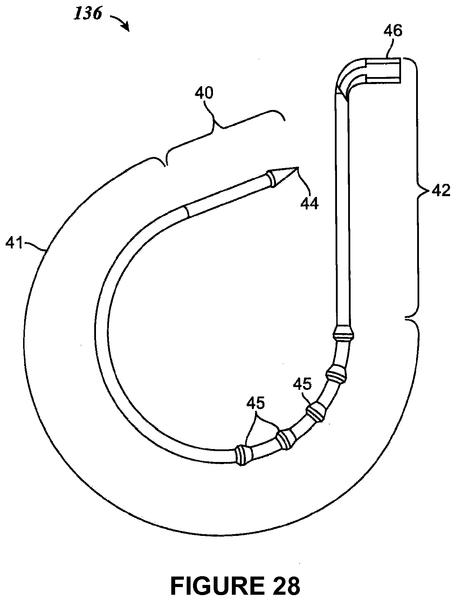

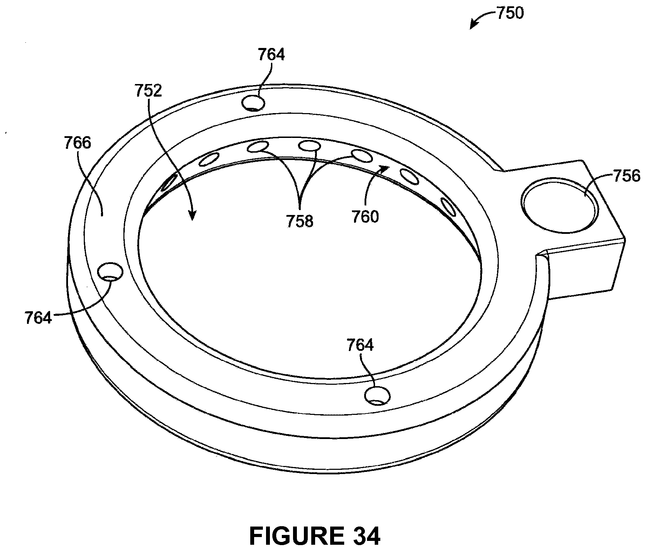

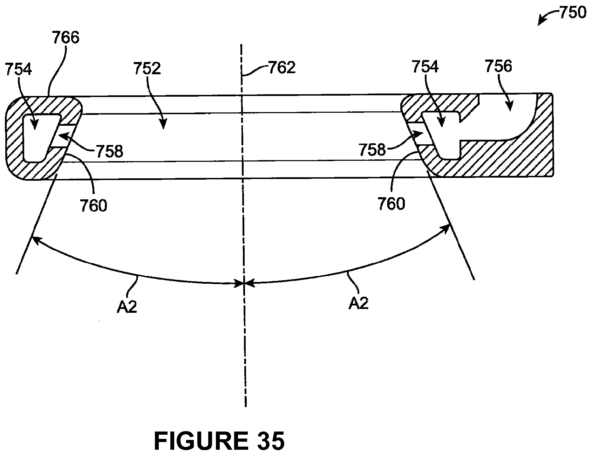

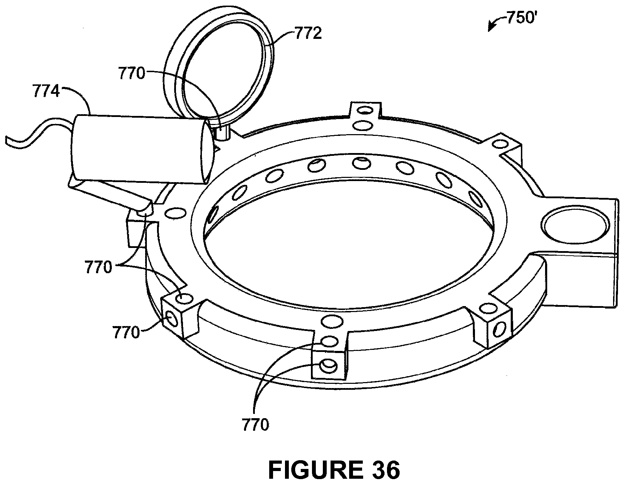

A second embodiment of a delivery tool is described below in connection with FIGS. 20-36. This embodiment shows delivery tool 111 which is similar to delivery tool 29, except that delivery tool 111 is operated through the use of hand actuated levers which drive and retract a needle (also called a "clip"), instead of using air pressure. Although delivery tool 111 is described in connection with a solid (not hollow) needle or clip 136, it is to be understood that delivery tool 111 can be adapted to carry attachment ring 30 of FIGS. 4 and 5 and suture device 28 of FIG. 1 and adapted to cinch suture device 28 to secure attachment ring 30 onto tissue. In such an adaptation, needle 24 may be modified to include catch 46 (FIG. 28) to allow delivery tool 111 to push needle 24 out of delivery tool 111 and into tissue. Delivery tool 111 pulls crimp 2 of suture device 28 during a cinching process. Hollow needle 24 of FIGS. 9 and 14-16, when in its natural, unconstrained state, can have the same actuate shape along its axial length as clip 136 of FIG 26. Delivery tool 111 includes contact members 702 which stabilize the tissue relative to delivery tool 111, as describe below. Delivery tool 29 of FIG. 6 can be modified to include contact members 702 and associated fluid conduits 704, which would surround vacuum cup 14. Also, vacuum cup 14 can be modified to have the configuration of ring-shaped contact member 750 (FIGS. 34 and 35) or 750' (FIG. 36).

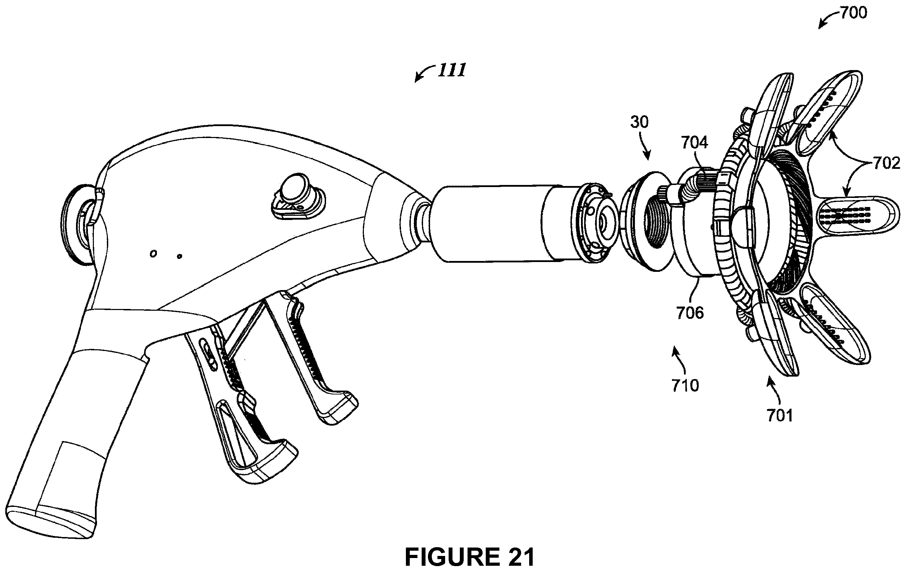

FIGS. 20 and 21 show delivery tool 111 for anchoring attachment ring 30 to biological tissue. Attachment ring 30 is a type of anastomotic prosthesis suitable for implantation within a human or animal body. Attachment ring 30 is a coupling for a conduit, graft, or other structure that is to be connected to a hollow anatomical organ. Forward segment 112 of delivery tool 111 is configured to engage attachment ring 30. Rear segment 114 has grip 116. Clip deployment handle 118, cinching handle 122, release knob 123, and disengagement knob 124 are used to control various elements in forward segment 112.

Attachment ring 30 has bottom end 132 and top end 134. Bottom end 132 is secured to biological tissue and top end 134 is configured to engage forward segment 112 of delivery tool 111. In some embodiments, attachment ring 30 includes features configured to connect with a conduit, such as an inflow conduit of a ventricular assist device (VAD), after attachment ring 30 has been secured to biological tissue, such as the ventricular apex of the heart. Methods for securing an inflow conduit to the ventricular apex by means of an attachment ring are described in U.S. Application Publication Nos. 2011/0118766 A1, 2011/0118833 A1, and 2011/0118829 A1.

In use, attachment ring 30 is engaged to and carried by connector mechanism 126 at forward segment 112 of delivery tool 111. The user positions forward segment 112 at the desired location on biological tissue where attachment ring 30 is to be secured. The user actuates the various controls on delivery tool 111 to simultaneously deploy multiple clips, or other type of anastomotic securements, that secure attachment ring 30 to the tissue and to disconnect connector mechanism 126 from attachment ring 30.

In some embodiments, when the user actuates clip deployment handle 118, clips are pushed out of delivery tool 111 such that the tips of clips pass through attachment ring 30 and into underlying tissue. The tips initially move downward in a substantially straight trajectory into the tissue. Thereafter, each tip follows a curved trajectory that extends radially outward and away from attachment ring 30 and returns upward out of the tissue. The tips then turn downward and return toward attachment ring 30 and stop at a position adjacent to or on an outer surface of attachment ring 30. Thus it will be understood that during deployment out of delivery tool 111, the clips pass through and curl back toward attachment ring 30. As the user continues to pull clip deployment handle 118, a portion of attachment ring 30 clamps down onto or traps the tips, and prevents the tips from moving backwards into the tissue. Next, as the user actuates cinching handle 122, the rear segment of each clip is pulled up away from the tissue and into delivery tool 111. Since the tip of each clip is held in place by attachment ring 30, pulling the rear segment of each clip causes the middle segment of each clip to cinch or tighten against the tissue. This tightening of the clips increases engagement between the tissue and attachment ring 30. When the user actuates release knob 123, the rear segment of each clip is released from delivery tool 111. Features on each clip and attachment ring 30, such as protrusions and catch features, prevent the rear segment of each clip from slipping or moving down toward the tissue, which maintains the cinched or tightened state of the clips. When the user actuates disengagement knob 124, attachment ring 30 is released by connector mechanism 126 of delivery tool 111, which allows delivery tool 111 to be pulled away from the tissue while attachment ring 30 remains attached to the tissue.

In use, engagement device 700 is fitted around and engaged to forward segment 112 of delivery tool 111. Engagement device 700 is configured to temporarily engage forward segment 112 of delivery tool 111 to biological tissue to facilitate accurate positioning of attachment ring 30 to the tissue and to facilitate deployment of clips into the tissue. Engagement device 700 is configured to selectively engage onto and disengage from the tissue with the application and removal of suction. Engagement device 700 is set to engage onto tissue, with application of suction, when clips are deployed and cinched by delivery tool 111. Engagement device 700 is set to disengage from the tissue, with a partial decrease or complete removal of suction, after the clips are deployed and before delivery tool 111 is pulled away from the tissue and attachment ring 30.

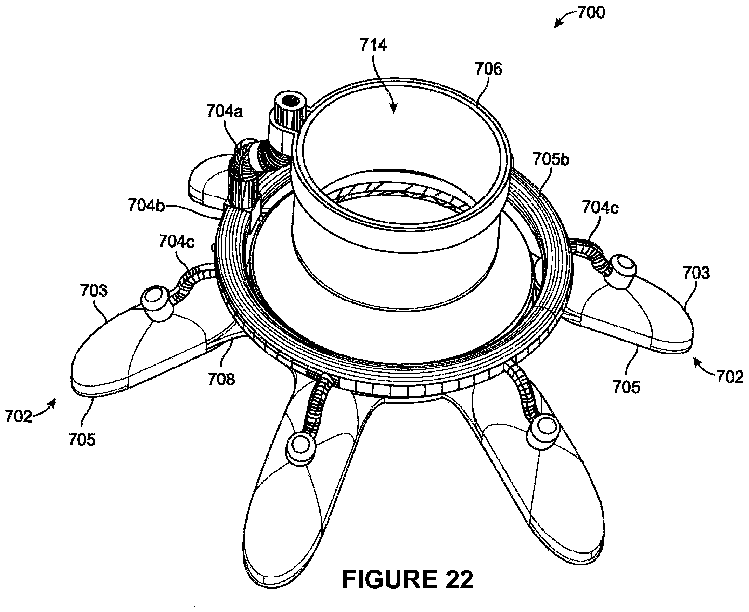

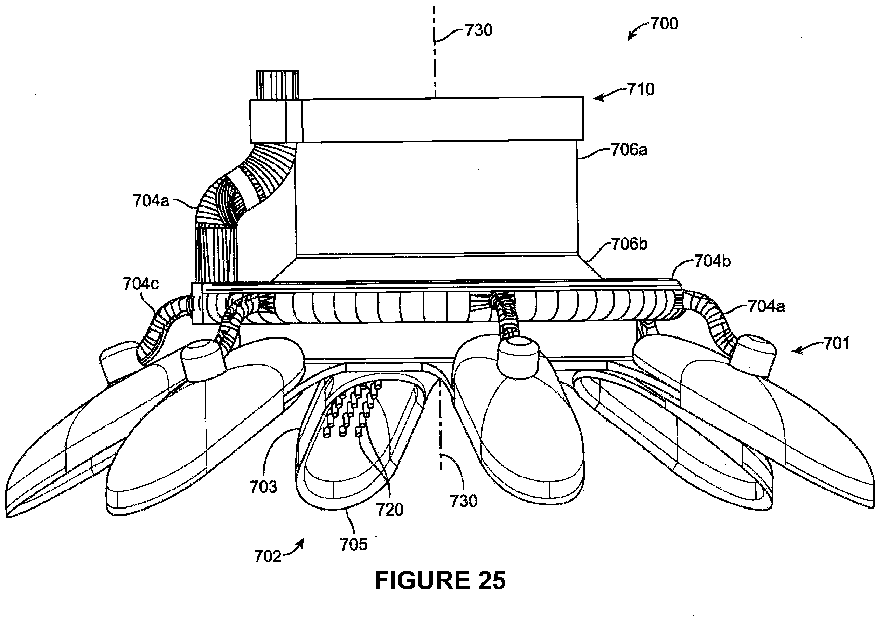

As shown in FIGS. 22-27 engagement device 700 includes a plurality of contact members 702, a fluid conduit 704, and coupling member 706. Contact members 702 are disposed at bottom or forward end 701 (FIG. 25) of engagement device 700. Each contact member 702 includes concave wall 703 forming a cup-shape. Concave wall 703 becomes thinner in thickness toward peripheral edge 705. Peripheral edge 705 of concave wall 703 is configured to conform to curvature at the surface of biological tissue. Each contact member 702 is attached by flexible joint 708 to coupling member 706 to accommodate tissue surface curvature. Concave wall 703 and joint 708 can be made of silicone rubber or other resilient material. In use, peripheral edges 705 will seal against biological tissue to maintain negative pressure and suction within the interior cavity enclosed by concave walls 703 of each contact member 702.

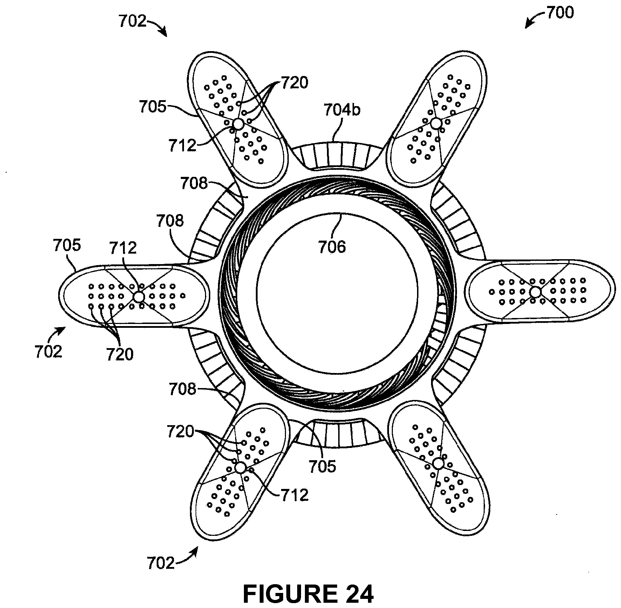

Fluid conduit 704 is a tube that extends from top or rear end 710 (FIG. 25) of engagement device 700, and connects to suction aperture 712 (FIG. 24) formed through concave walls 703 of each contact member 702. Fluid conduit 704 is configured to convey suction to and draw air out of the interior cavity of each contact member 702.

Fluid conduit 704 includes rear tube 704a, ring tube 704b, and a plurality of forward tubes 704c. Rear tube 704a is configured to be connected to a negative pressure source or suction pump. An end of rear tube 704a is connected to ring tube 704b which is connected to ends of forward tubes 704c. The opposite end of forward tubes 704c are connected to suction apertures 712 (FIG. 24) in concave walls 703 of contact members 702. The lumen of rear tube 704a, ring tube 704b, and forward tubes 704c are interconnected so that any negative pressure, vacuum, or suction applied to rear tube 704a is fluidly communicated to the interior cavity of each contact member 702. When peripheral edges 705 of contact members 702 are disposed on the surface of tissue, contact members 702 engage and maintain hold of the tissue so as to prevent or minimize relative movement between delivery tool 111 and the tissue.

Coupling member 706 is in the shape of a tube and has central lumen 714. Central lumen 714 is sized to receive forward segment 112 of delivery tool 111. Six contact members 702 are arranged circumferentially around central lumen 714 at substantially equal circumferential spacing of about 60 degrees. In other embodiments, an engagement device can include a lesser or greater number of contact members 702.

Referring to FIGS. 25-27, coupling member 706 includes narrow portion 706a at rear end 710, flared portion 706b, and wide portion 706c at forward end 701. Flared portion 706b extends radially outward from narrow portion 706a. Flared portion 706b connects narrow portion 706a to wide portion 706c. In use, narrow portion 706a is attached to forward segment 112 of delivery tool 111. Narrow portion 706a, flared portion 706b, and wide portion 706c can be formed of a substantially transparent or translucent material, such as a clear polycarbonate polymer, to allow the user to see underlying biological tissue and placement of attachment ring 30 on the tissue.

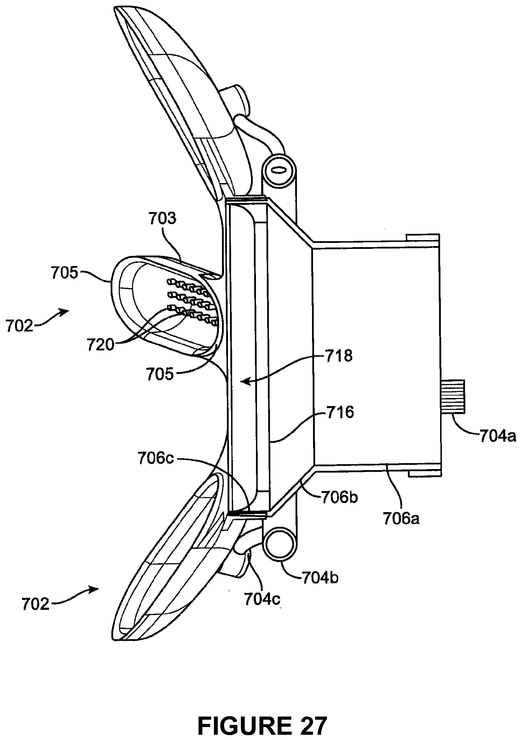

As shown in FIGS. 26 and 27, the exterior surface of wide portion 706c is attached by flexible joints 708 to contact members 702. The interior surface of wide portion 706c is attached to guide ring 716. Guide ring 716 is disposed within wide portion 706c. Guide ring 716 includes a smooth, annular surface 718 that slopes and curves radially inward as it extends rearward away from forward edge 706d of wide portion 706c. Annular surface 718 helps to insure radial inward travel of tips of securement that are deployed out of delivery tool 111.

As shown in FIGS. 24-27, each contact member 702 includes a plurality of protrusions 720 in the form of cylindrical posts attached to the interior surface of concave walls 703. Protrusions 720 surround suction aperture 712 and can help maintain a large area of suction and/or increase frictional engagement with biological tissue.

In some embodiments, concave walls 703 may be made of a flexible material, such as silicone rubber, to facilitate formation of a seal between peripheral edge 705 and the surface of tissue. As negative pressure builds in the cavity between concave walls 703 and the tissue surface, concave walls 703 may tend to collapse toward the tissue surface. Such collapse may result in deformation of peripheral edges 705 that causes loss of the air seal between peripheral edges 705 and the tissue. Also, if suction aperture 712 contacts tissue surface, suction and thus the area of tissue surface engagement will be limited to the area immediately surrounding suction aperture 712. Protrusions 720 are configured to help maintain the air seal and to prevent suction aperture 712 from contacting the tissue surface so that the area of tissue surface engagement extends out to peripheral edges 705 of contact members 702.

In some embodiments, protrusions 720 are configured to provide frictional engagement against the tissue surface. As negative pressure builds in the cavity between concave walls 703 and the tissue surface, concave walls 703 may tend to collapse toward the tissue surface so that protrusions 720 presses against and frictionally engage the tissue surface.

The clips for anchoring attachment ring 30 to biological tissue are contained within forward segment 112 of delivery tool 111. The configuration of each clip can be as shown in FIG. 28. Clip 136 (also referred to as a needle) is formed from a metal wire made of a nickel-titanium alloy, Nitinol, or other material having shape memory and/or superelastic properties. Clip 136 includes forward segment 40 and rear segment 42, both of which are substantially straight. Forward segment 40 has sharp tip 44 for piercing a portion of attachment ring 30 and underlying biological tissue. Catch 46 protrudes out from rear segment 42 and is pushed forward (deployment) and pulled backward (cinching) during operation of delivery tool 111. Curved segment 41 connects forward segment 40 to rear segment 42. Curved segment includes a plurality of studs or bumps 45 that protrude radially outward. After clips 136 are cinched, the bumps 45 prevent the clips moving forward due to tension. Each bump 45 allows for a different amount of cinching to accommodate variations in the thickness of biological tissue. Bumps 45 can be shaped and sized to engage one or more structural catch features of attachment ring 30 to inhibit or prevent clip 136 from loosening after being cinched.

When loaded inside delivery tool 111, curved segment 41 is in a straightened configuration. When deployed out of delivery tool 111, tip 44 will initially follow a straight path through attachment ring 30 and into the biological tissue. As curved segment 41 exits delivery tool 111, curved segment will autonomously return to a curved configuration, which causes tip 44 to follow a curved path beneath the tissue surface. Due to the curved path, tip 44 exits the tissue surface and loops back toward attachment ring 30. Attachment ring 30 includes features that clamp and/or trap tip 44. As tip 44 exits the tissue surface, annular surface 718 (FIGS. 26 and 27) on engagement device 700 may help guide tip 44 toward attachment ring 30.

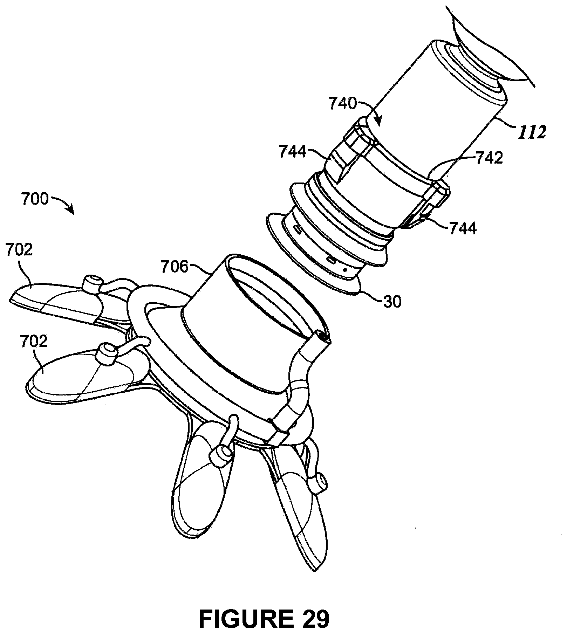

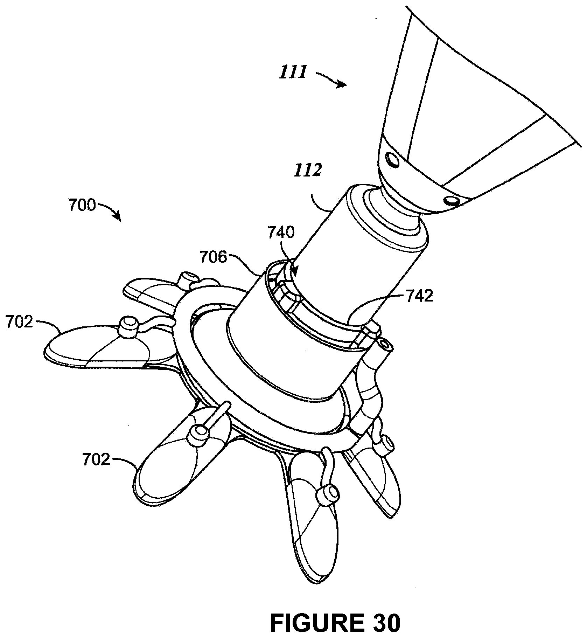

FIGS. 29 and 30 show connector 740 for connecting engagement device 800 to forward segment 112 of delivery tool 111. Connector 740 includes a circular band 742 that wraps around forward segment 112. Barbed arms 744 protrude axially from band 742 and are configured to engage a ring feature within coupling member 706 of engagement device 800. When connector 740 engages and retains engagement device 700, attachment ring 300 is at the appropriate position relative to the suction provided by contact members 702 during use. As will be discussed below, the relative position of attachment ring 300 and contact members 702 is controlled so that force loading of the heart tissue does not damage the tissue while allowing clips to be properly deployed.

In some embodiments, band 742 of connector 740 is fixedly attached to forward segment 112 of delivery tool 111 with no ability to adjust the axial position of contact members 702 of engagement device 700 relative to forward segment 112 and attaching ring 30. In other embodiments, band 742 is slideably attached to forward segment 112 to allow a medial practitioner to selectively adjust and fix the axial position of contact members 702 relative to forward segment 112 and attachment ring 30.

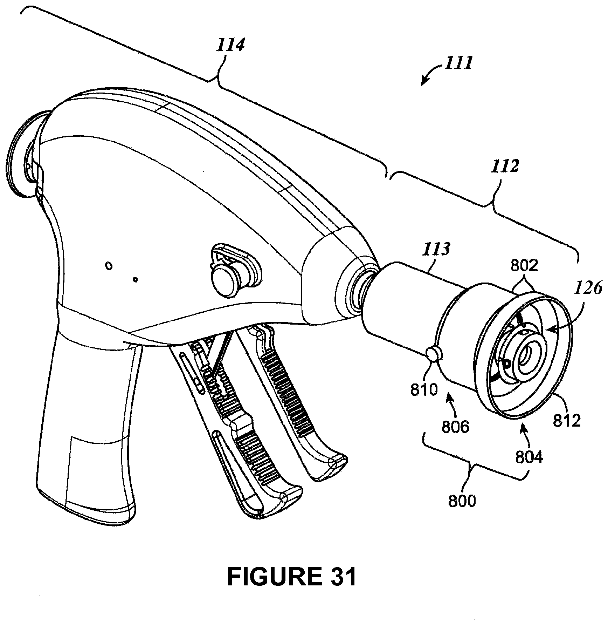

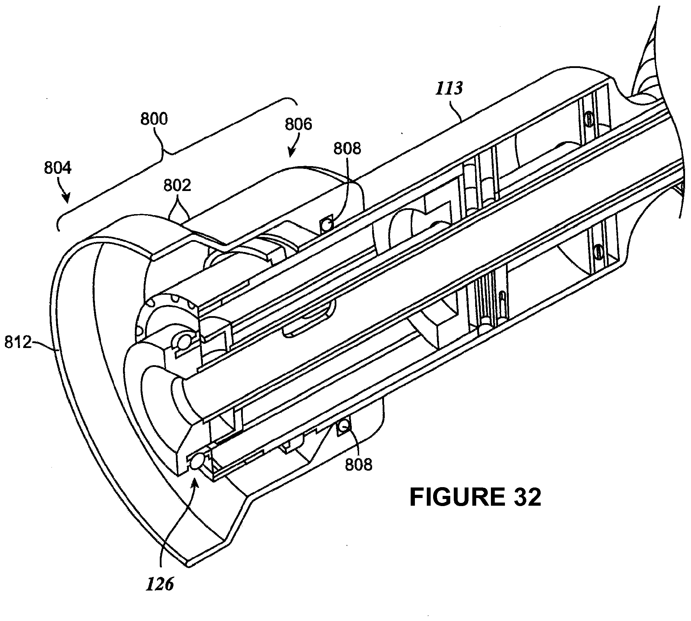

FIGS. 31 and 32 show delivery tool 111 of FIG. 20 with engagement device 800. Engagement device 800 includes suction cap 802 configured to maintain suction over the surface of tissue. Suction cap 802 is tubular in shape and comprises forward end 804 and rear end 806. Rear end 806 is slideably coupled to forward segment 112 of delivery tool 111. O-ring gasket 808 maintains a substantially fluid-tight seal between suction cap 802 and outer surface 113 of delivery tool 111. Set screw 810 is located on suction cap 802 to selectively allow and prevent sliding of suction cap 802. The head of the set screw 810 is exposed while the tip of set screw 810 is disposed adjacent outer surface 113 of delivery tool 111.

In use, attachment ring 30 (not shown in FIGS. 31 and 32) is carried on connector mechanism 126 of delivery tool 111 and within suction cap 802. The user maneuvers delivery tool 111 so that forward edge 812 of suction cap 802 is placed on the tissue surface. Forward edge 812 is circular and has an inner diameter larger than the outer diameter of attachment ring 30. Forward edge 812 is configured to seal against biological tissue to maintain negative pressure and suction within the interior cavity of suction cap 802. To accommodate a variety of possible curvatures in the biological tissue, the user may slide suction cap 802 axially on forward segment 112 of delivery tool 111 so that attachment ring 30 and forward edge 812 of suction cap 802 simultaneously contact the tissue surface. When suction cap 802 is at the desired axial position on forward segment 112, the user can manipulate the screw head so that the tip of set screw 810 presses against forward segment 112 and locks the position of suction cap 802. The user can apply a vacuum or suction through delivery tool 111 to produce negative pressure within the interior cavity of suction cap 802. With the negative pressure, forward edge 812 of suction cap 802 engages the tissue surface and prevents or minimizes relative movement between delivery tool 111 and the tissue surface. Since attachment ring 30 is located within suction cap 802, the tissue surface directly below attachment ring 30 is held in place against attachment ring 30.

In other embodiments, engagement device 800 is combined with all the features of engagement device 700 such that coupling member 706 is replaced by suction cap 802. Alternatively, coupling member 706 can be fitted over and attached onto suction cap 802. Suction can be applied to contact members 702 and/or suction cap 802 to engage delivery tool 111 to tissue. Applying suction to contact members 702 helps to stabilize areas of tissue spaced apart from and surrounding attachment ring 30. Applying suction to suction cap 802 helps to stabilize areas of tissue directly beneath attachment ring 30. While negative pressure is generated within contact members 702 and/or suction cap 802, clips 136 are deployed out of delivery tool 111 and into the underlying tissue.

During a surgical procedure, engagement device 700, 800 and delivery tool 111 are fixed to each other. Delivery tool 111 is hand held by the surgeon who manipulates controls on the delivery tool. As the surgeon places delivery tool 111 at the target tissue, engagement device 700, 800 will engage surrounding tissue with application of suction and thereby prevent or minimize relative movement between delivery tool 111 and the target tissue. Engagement device 700, 800 is not fixedly connected to a surgical bed, sternum retractor, or other stationary structure. Engagement device 700, 800 does not fix or stabilize the surrounding tissue relative to a stationary structure. Engagement device 700, 800 fixes or stabilizes the surrounding tissue relative to delivery tool 111, which moves under the direction of the clinician. Thus, after engagement device 700, 800 attaches to surrounding tissue with suction, and if the clinician manually moves delivery tool 111 left, right, up or down within the patient's body cavity, delivery tool 111 will remain stationary relative to the target tissue. Delivery tool 111 will also remain at the target tissue even if the target tissue moves due to pulsatile blood flow, unexpected change in the patient's body position, or other reason. Thus it will be understood that delivery tool 111 and engagement device 700, 800 are designed to move with the target tissue and not to slip relative to the target tissue. Engagement device 700, 800 enables delivery tool 111 to be "stuck" with the target tissue, which helps to deploy the clips at the precise location selected by the surgeon. If desired, suction can be partially decreased or completely removed to allow the surgeon to adjust the position of delivery tool 111 on the tissue.

As discussed above, negative pressure or suction is applied to the engagement device 700, 800 so that engagement device 700, 800 attaches to the target tissue. A particular amount of negative pressure is necessary for the purpose of deploying the clips with consistency from delivery tool 111. Inconsistent clip deployment may take the form of undue variation in depth, direction, and/or curvature in the path of travel of the clips. The amount of negative pressure needed for consistent clip deployment may depend on multiple factors, including but not limited to the number of clips being deployed, the type of tissue into which the clips are being deployed, and the size of the surface area to which vacuum is being applied. A method according to the invention can include the steps discussed above in combination with either one or both of (1) the step of selecting or determining an amount of negative pressure that corresponds to a threshold level of security between the delivery tool and the target tissue, and (2) the step of applying the preselected or predetermined amount of negative pressure to the engagement device 700, 800 during clip deployment. In various embodiments, the determined amount of negative pressure is selected to be below a maximum threshold. The maximum threshold may be selected to reduce the risk of injury to the target tissue.

Engagement device 700, 800 can be used for locating and targeting the site for attaching a VAD inflow conduit during an off-pump, minimally invasive surgical procedure as opposed to a conventional, open heart procedure. The term "off-pump" means that clip deployment is performed while the heart is beating and without a heart-lung or cardiopulmonary bypass procedure being performed. For example and without limitation, delivery tool 111 and engagement device 700, 800 can be introduced into the chest cavity of a patient via an intercostal approach or via a small incision between the ribs of the patient. In this example, a sternotomy and spreading of the left and right rib cage apart are avoided. After introduction into the chest cavity, the clinician can place delivery tool 111 on the ventricular apex of the heart (the target tissue) and apply a sufficient amount of negative pressure to engagement device 700, 800 that substantially prevents relative movement between the target tissue and delivery tool 111.

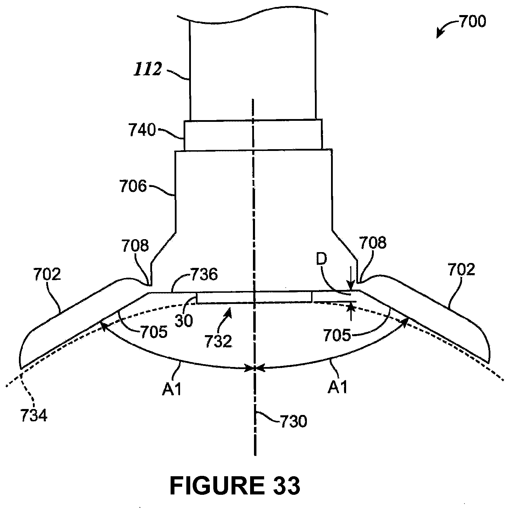

Engagement device 700, 800 applies preset or adjustable force loading between the delivery tool 111 and the tissue. This loading is important for the success of deploying the clips. When delivery tool 111 is used in concert with engagement device 700, 800, and as a vacuum is applied, delivery tool 111 is pressed downward (as a result of the suction) against the tissue. The downward force (also referred to as load) applied by delivery tool 111 on the tissue depends upon a connection device that connects engagement device 700, 800 and delivery tool 111 together. The exemplary connection device allows for control of the amount of force on the tissue so that the force is simultaneously (1) below a maximum level that would damage the tissue, and (2) at or above a minimum level needed to ensure that the clips are properly deployed without undue variation in depth, direction, and/or curvature in the path of travel of the clips. Control of the amount of force on the tissue can be accomplished by adjusting the distance between the forward tip of attachment ring 30 and the base (736 in FIG. 33, or 812 of FIG. 31) of engagement device 700, 800. The amount of force is increased when the attachment ring 300 is placed at a greater distance distal to or in front of the base of engagement device 700, 800. The connection device, for adjusting the distance, can include connector 740 (FIGS. 29 and 30) and/or screw 810 (FIG. 31).

Engagement device 700, 800 keeps the deployment site clear of possible obstruction. Engagement device 700, 800 completely engages the tissue area required for clip deployment and prevents surrounding tissue and other surgical devices from entering into that tissue area and interfering with clip deployment.

Engagement device 700 has contact members 702 that function as individual pods or feet that provide suction to discrete areas of tissue surrounding a central area into which clips are deployed. The central area is referred to as the clip deployment site. The discrete areas of tissue can be separated from each other by areas of tissue which receive no suction. Engagement device 800 has suction cap 802 that provides suction to a circular area that includes the clip deployment site.

As shown in FIG. 25, each contact member 702 has peripheral edge 705 that forms the boundary of a suction port or opening. In various embodiments, each contact member has a peripheral edge shaped and configured to improve sealing for applying suction. The suction openings face inward toward central axis 730 of engagement device 700. Contact members 702 can be symmetrically arranged around central axis 730. In use, the suction openings face inward toward attachment ring 30 (not shown in FIG. 25). As shown in the partial, cross-sectional diagram of FIG. 33, suction openings face inward toward clip deployment site 732 and are oriented at acute Angle A1 relative to central axis 730. Due to Angle A1, the suction openings of contact members 702, as a group, create a frustoconical shape or a cup shape that rests on the curved outer surface of the ventricular apex of the heart during a surgical procedure.

As shown in FIG. 33, contact members 702 extend radially outward and in a forward direction from base 736 of coupling member 706 of attachment device 700. Attachment device 700 is secured to forward segment 112 of delivery tool 111 such that attachment ring 30, which is held by forward segment 112, protrudes in a forward direction from base 736 by Axial Distance D. Attachment device 700 is secured to forward segment 112 such that, during clip deployment, Axial Distance D results in a loading force on the heart tissue that is simultaneously (1) below a maximum level that would damage the tissue, and (2) at or above a minimum level needed to ensure that the clips are properly deployed without undue variation in depth, direction, and/or curvature in the path of travel of the clips. In other embodiments, attachment ring 30 is level with base 736 during clip deployment. In other embodiments, attachment ring 30 is located below base 736 so as to be recessed within coupling member 706 during clip deployment.

The orientation and shape of the suction ports serve multiple purposes. Angle A1 is selected to allow for easy attachment of engagement device 700 to the heart. Angle A1 allows engagement device 700 to conform to the curved outer surface 734 of the heart. Angle A1 can be within the range of about 10 degrees to about 80 degrees, or more narrowly within the range of about 20 degrees to about 70 degrees, or at about 60 degrees. The appropriate angle depends in part on the physical size of the patient's heart. A relatively small angle would be more suitable for the curved outer surface of a relatively small heart, and a relatively large angle would be more suitable for the curved outer surface of a relatively large heart.

The orientation and shape of the suction openings will remodel the clip deployment site for engagement with delivery tool 111 and attachment ring 30. The configuration of the suction openings will pull the heart tissue 734 up to form an ideal shape for engagement with delivery tool 111 and attachment ring 30. The slanted angle of the engagement device surface in contact with the heart tissue 734, in combination with the vacuum suction, will change the shape of the heart so it can conform to that of the engagement device and/or attachment ring 30.

The orientation and shape of the suction openings minimize the movement of the tissue during clip deployment.

The angular orientation of the suction opening, Angle A1, can be adjusted according to the surgeon's need. Contact members 702 are adjustable and designed to contour to the shape of the outer surface of the heart. Making joint 708 (FIG. 25) from a flexible and elastic material allows for adjustment.

An engagement device can have an annular or ring-shaped contact member 750, as shown in FIGS. 34 and 35. Ring-shaped contact member 750 can be implemented as an alternative to or in combination with contact members 702 and/or suction cap 802. Contact member 750 is hollow and substantially circular, with a central opening 752 sized to receive coupling member 706 (FIG. 21) of the engagement device and attachment ring 30 carried by delivery tool 111. An interior air passageway 754 encircles central opening 752. Passageway 754 is in fluid communication with outlet port 756 and a plurality of inlet ports, also referred to as suction openings 758. Air is drawn out of passageway 754 from outlet port 756. Outlet port 756 can be connected to a vacuum source, such as rear tube 704a in FIG. 122. Air is drawn into passageway 754 from suction openings 758, which are configured to apply suction to heart tissue. Suction openings 758 are spaced apart from each other and are circumferentially distributed around central opening 752. Suction openings 758 are formed into interior surface 760 of contact member 750. In various embodiments, interior surface 760 is frustoconical in shape. The exemplary interior surface faces radially inward toward central opening 752 and central axis 762 of contact member 750.

Interior surface 760 is oriented at acute Angle A2 relative to central axis 762. Angle A2 can be about 25 degrees. In other embodiments, Angle A2 can be within the range of about 10 degrees to about 80 degrees, or more narrowly within the range of about 20 degrees to about 70 degrees, or at about 60 degrees. Angle A2 is selected with the purpose of maintaining contact with the heart surface which is not flat, but curved so that ring-shaped contact member 750 can "cup" the heart.

Contact member 750 includes multiple attachment points 764 (FIG. 34) on an exterior, rearward facing surface 766. Attachment points 764 allow contact member 750 to be retained and secured by a connector on forward segment 112 of delivery tool 111. The connector can be the same as or similar to connector 740 described above in connection with FIGS. 29 and 30.

As shown in FIG. 36, ring-shaped contact member 750' includes a plurality of accessory attachment points 770, in addition to all the features for contact member 750 of FIGS. 34 and 35. Accessory attachment points 770 are configured to receive and retain accessories, including without limitation adjustable mirror 772 and adjustable light 774 for improving visibility during a surgical procedure. Accessory attachment points 770 are distributed circumferentially to allow a variety of mounting positions for the accessories.

In other embodiments, engagement device 700, 800 has attachment points around the outer perimeter of the device. The attachment points are configured to receive and retain add-on devices, such as a viewing port or a light source. The attachment points can be the same as or similar to accessory attachment points 770 described in connection with FIG. 36.

### THIRD EMBODIMENT

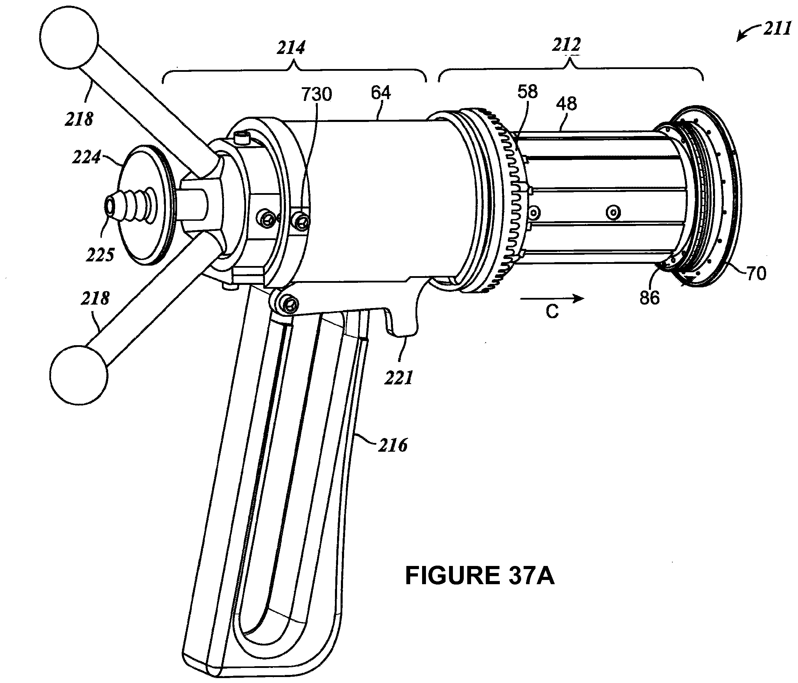

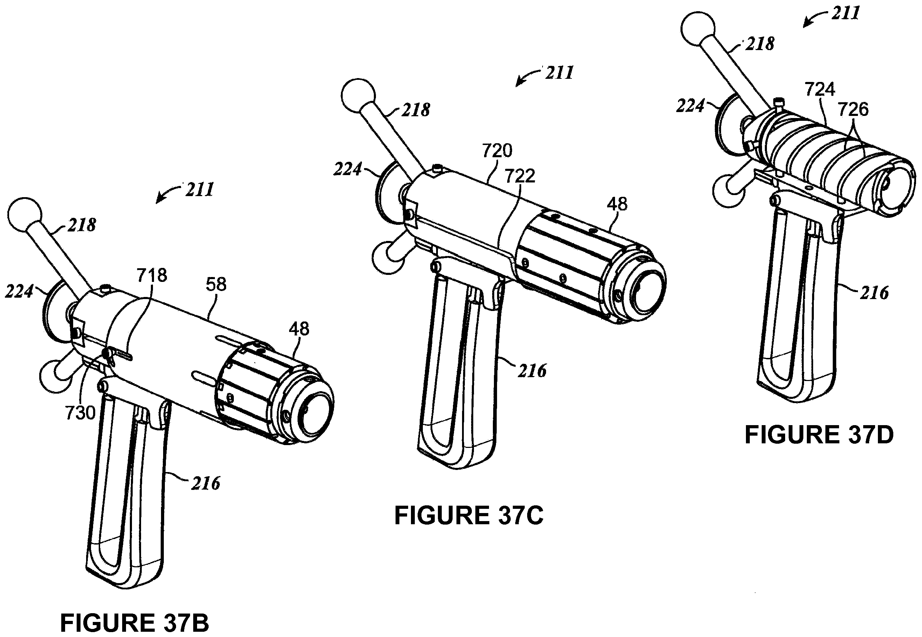

A third embodiment of a delivery tool is described below in connection with FIGS. 37A-37E. This embodiment shows delivery tool 211 which is similar to delivery tool 29, except that delivery tool 211 is operated through the use of movable grip 16 and handle 18 to drive and retract a needle (also referred to as a clip), instead of using air pressure. Although delivery tool 211 is described below in connection with a solid (not hollow) clip 136, it is to be understood that delivery tool 211 can be adapted to carry attachment ring 30 of FIGS. 4 and 5 and suture device 28 of FIG. 1 and adapted to cinch suture device 28 to secure attachment ring 30 onto tissue. In such an adaptation, needle 24 may be modified to include catch 46 (FIG. 28) to allow delivery tool 211 to push needle 24 out of delivery tool 211 and into tissue. Delivery tool 211 pulls crimp 2 of suture device 28 during a cinching process.

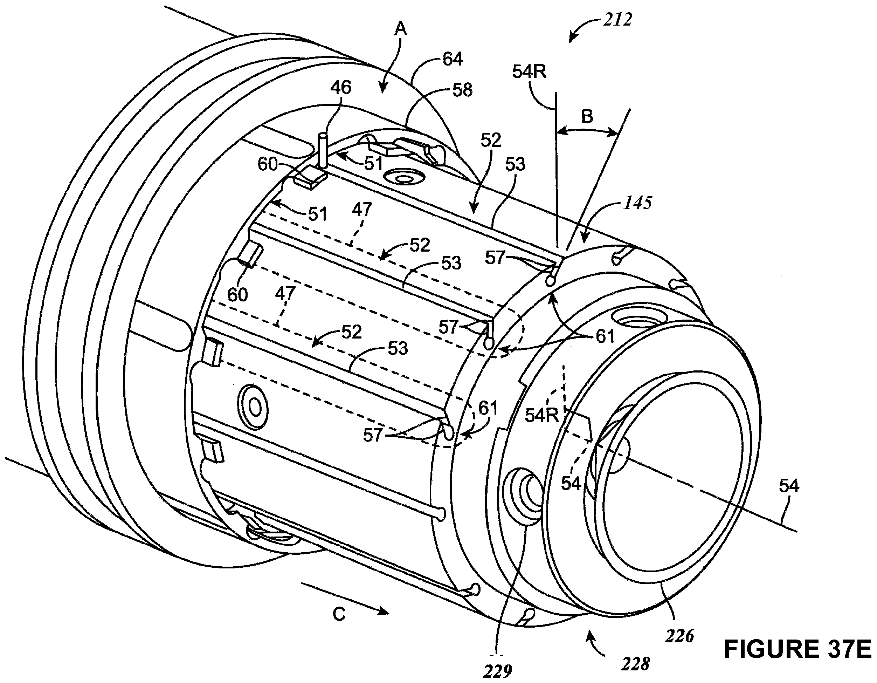

FIG. 37A shows delivery tool 211 for anchoring attachment ring 30 to biological tissue. Although attachment ring 30 is shown and described together with delivery tool 211, it will be appreciated that other delivery tools may be used to anchor attachment ring 30 to biological tissue. FIGS. 37B-37D shows delivery tool 211 without attachment ring 30 and in varying states of disassembly. FIG. 37E shows a detailed view of an exterior portion of delivery tool 211 on which attachment ring 30 could be carried. Exemplary attachment ring 30 is a type of prosthesis suitable for implantation within a human or animal body. Attachment ring 30 is a coupling for a conduit, graft, or other structure that is to be connected to biological tissue. In various embodiments, attachment ring 30 is configured for attaching a device (e.g. a prosthesis, therapy device, a diagnostic device, etc.) to a body lumen or organ. Forward segment 212 of delivery tool 211 is configured to engage attachment ring 30. Rear segment 214 has grip 216. Clip deployment handle 218, clamp release 221, and disengagement knob 224 are used to control various elements in forward segment 212. As described below, clip deployment handle 218 also provides clamping and cinching functions.

In use, clips 136 (also referred as needles) are contained within forward end 212 of delivery tool 211. Each clip 136 includes wire body having forward segment 40 and rear segment 42. Forward segment 40 has sharp tip 44 for piercing a portion of attachment ring 30 and underlying biological tissue. Catch 46 protrudes out from rear segment 42 and is pushed forward during operation of delivery tool 211. Clips 136 are constrained in a straightened configuration within forward end 212 of delivery tool 211. In various embodiments, the clips are formed of shape memory material and make use of the shape memory properties. When deployed out of forward end 212, exemplary clips 136 will autonomously coil radially outward away from axial centerline 54 (FIG. 37E) in a direction away from forward end 212 due to elastic memory of wire body 38. In various embodiments, the clips have a generally straight shape in a stowed or undeployed condition and a relatively curved shape when deployed. In various embodiments, at least a portion of the clips extend outwardly away from the forward end without the use of external forces when they are unconstrained. One will appreciate that the shapes and configurations of the clips in the deployed and undeployed conditions may be modified depending on the application. For example, the clips may have a relatively straighter shape when deployed.

Referring to FIG. 37E, clips 136 are constrained within a plurality of clip holders 47 forming parts of clip tube 48. Clip tube 48 is a hollow, cylindrical sleeve. Each clip holder 47 comprises clip groove 52 formed within walls of clip tube 48. Clip groove 52 has axial slot opening 53 that faces radially outward, away from axial centerline 54 of clip tube 48. An end portion of catch 46 of each clip 136 extends out of axial slot opening 53 of clip groove 52. One exemplary catch 46 is shown for ease of illustration, and it will be understood there will be a catch protruding out of each clip groove 52 that contains clip 136. Clip pusher surface 51 abuts catch 46 from behind and is configured to push clips 136 out of forward opening 61 of clip groove 52..

Clip grooves 52 have sidewalls 57 that extend substantially parallel to axial centerline 54 and substantially non-perpendicular to outer surface 145 of clip tube 48. In other embodiments, sidewalls 57 are substantially perpendicular to outer surface 145.

Catch 46 of each clip 136 abuts sidewalls 57 of clip groove 52, which prevents clip 136 from twisting about its central axis 39 while contained inside clip groove 52. Catch 46 and sidewalls 57 help to ensure that the curved trajectory of tip 44 will be in the desired direction relative to attachment ring 30. The direction followed by tip 44 is controlled in part by the angle of sidewalls 57 and by the initial shape of clip 136 prior to being loaded in delivery tool 211. As shown in FIG. 37E, sidewalls 57 are at an oblique angle measured from radial line 54R. Radial line 54R is a radial line that extends out from the center of clip tube 48 and is perpendicular to axial centerline 54. The oblique angle, indicated by arrow B, can be from about 211 degrees to about 80 degrees, and more narrowly from about 30 degrees to about 60 degrees, and more narrowly at about 45 degrees. In some embodiments, the angle of sidewalls 57 causes clips 136 to deploy into biological tissue at the oblique angle relative to radial line 54R. A change in oblique angle B changes the distance between the center of delivery tool 211 and the point at which the clip tip 44 exits the biological tissue, and thus changes the size of the clip foot print. Oblique angle B is important since clip tip 44 should exit the biological tissue at a point slightly beyond the outer circumference of attachment ring 30. A larger oblique angle B results in a smaller clip footprint and thereby increases hemostasis and stabilization of attachment ring 30 to the biological tissue. The term "clip footprint" refers to the surface area of biological tissue encircled by a plurality of deployed clips.

In FIG. 37E, oblique angle B is the same for sidewalls 57 of all clip grooves 52. In other embodiments, clip grooves 52 can have varying oblique angles. For example, a first group of clip grooves 52 at a first area of clip tube 48 have sidewalls 57 oriented at oblique angle B that is different than that of a second group of clip grooves 52 at a second area of clip tube 48. For example, on the same clip tube, oblique angle B can be 30 degrees for some clip grooves 52, and 45 degrees for other clip grooves, and 60 degrees for other clip grooves 52.

There are twelve clip holders 47 circumferentially arranged on clip tube 48 at substantially equal angular spacing of about 30 degrees apart from each other. In other embodiments, a fewer number or a greater number of clip holders 47 are arranged around the clip tube than what is shown in FIG. 37E. The number of clip holders and clips depends upon a variety of factors, such as the type of surgical procedure that is being performed and the type and condition of the biological tissue to which attachment ring 30 is to be anchored. In other embodiments, the clip holders are not arranged at equal angular spacing, such that the clip holders are closer to each other at one area of clip tube 48 as compared another area of the clip tube 48.

Cinching tube 58 is a hollow, cylindrical sleeve. Cinching tube 58 contains and is substantially coaxial with clip tube 48. Clip pusher surface 51 (FIG. 37E) is located at the forward end of cinching tube 58. Cinching pins 60 are attached to cinching tube 58 and protrude axially in front of clip pusher surface 51. Cinching tube 58 is controlled by clip deployment handle 218 (FIG. 37A). Clamping tube 64 is a hollow, cylindrical sleeve. Clamping tube 64 contains and is substantially coaxial with clip tube 48 and cinching tube 58. Clamping tube 64 is controlled by handle 218.

A method for anchoring attachment ring 30 will now be described together with delivery tool 211, though it should be understood that other delivery tools may be used to perform the method. It is to be understood that, depending on the type of delivery tool used and depending on clinical need, some steps described below may be performed simultaneously as a single step, performed in a sequence other than described below, or may be omitted.

Exemplary steps for delivery tool stabilization are as follows. Referring to FIG. 37A, a user such as a medical practitioner grasps grip 216 to position main body 70 of attachment ring 30 over biological tissue. Suction may be applied to tube fitting 225 which conveys the suction to suction cup 226 (FIG. 37E) at the front of delivery tool 211. Suction cup 226 engages the biological tissue and stabilizes delivery tool 211 against movement relative to the biological tissue. Steps for stabilization can be performed whenever needed during the process of deploying clips 136 into attachment ring 30 and tissue.

Exemplary steps for clip deployment are as follows. The user rotates handle 218 to begin deployment of clips 136 out of delivery tool 211. Handle rotation causes clamping tube 64 (FIG. 37A), cinching tube 58, and clamping ring 100 to slide axially forward onto clip tube 48 in the direction of arrow C. Forward end of cinching tube 58 has clip pusher surface 51 (FIG. 37E) that pushes clips 136 out of delivery tool 211, through cinching ring 86 and attachment ring main body 70, and into the biological tissue. As clip pusher surface 51 continues to push rear segment 42 of clips 136, sharp tips 44 of clips 136 follow a curved path into and then out of the biological tissue.

The foregoing descriptions of specific embodiments of the present invention have been presented for purposes of illustration and description. They are not intended to be exhaustive or to limit the invention to the precise forms disclosed, and obviously many modifications and variations are possible in light of the above teaching. The embodiments were chosen and described in order to best explain the principles of the invention and its practical application, to thereby enable others skilled in the art to best utilize the invention and various embodiments with various modifications as are suited to the particular use contemplated.

## Claims

1. A suture device (28), comprising:
a suture core (1) having a core inner lumen (101);
a suture crimp (2) positioned at a proximal end of the suture core, the suture crimp (2) fastened to a suture (102) within the core inner lumen (101); and
an anchoring sleeve (3) positioned at a distal end of the suture core (1),
wherein the anchoring sleeve (3) is compressible in an axial direction, is secured to a distal-most end of the suture core (1), and extends in a proximal direction, and wherein the anchoring sleeve (3) is adapted to collapse and anchor the distal end of the suture core (1) against a tissue wall.

2. The suture device (28) of claim 1, wherein the suture crimp (2) is mechanically crimped on the suture core (1).

3. A suture assembly, comprising:
the suture device (28) according to claim 1 or 2; and
a tubular needle (24) including a needle inner lumen adapted to receive the suture core (1);
wherein the suture crimp (2) is adapted and dimensioned to abut a proximal end of the needle.

4. The suture assembly of claim 3, wherein the tubular needle (24) has a predefined shape.

5. The suture assembly of claim 4, wherein the tubular needle (24) is formed of a shape memory alloy.

6. The suture assembly of claim 4, wherein the tubular needle (24) is formed of nitinol.

7. The suture assembly of claim 4, wherein the tubular needle (24) is formed of a superelastic material.

8. An anastomotic system, comprising:
a suture device according to claim 1; and a tubular needle including a needle inner lumen for receiving the suture core; and
a delivery tool for deploying the tubular needle (24).

9. The system of claim 8, wherein the tubular needle has a predefined shape.

10. The system of claim 8, wherein the tubular needle has a predefined arcuate shape.

11. The system of claim 9 or 10, wherein the tubular needle is formed of a shape memory alloy.

12. The system of claim 9 or 10, wherein the tubular needle is formed of nitinol.

13. The system of any one of claims 8 to 12, further comprising a plurality of tubular needles and a plurality of suture devices, each suture device received within a needle inner lumen of a respective needle.

## Patentansprüche

1. Eine Nahtvorrichtung (28), umfassend:
einen Nahtkern (1), der ein inneres Kernlumen (101) hat;
eine an einem proximalen Ende des Nahtkerns angeordnete Nahtkräuselung (2), wobei die Nahtkräuselung (2) an einem Naht (102) innerhalb des inneren Kernlumens (101) befestigt ist; und
eine an einem distalen Ende des Nahtkerns (1) angeordnete Ankerhülse (3), wobei die Ankerhülse (3) in einer axialen Richtung komprimierbar ist, an einem am distalsten gelegenen Ende des Nahtkerns (1) befestigt ist und sich in einer proximalen Richtung erstreckt, und wobei die Ankerhülse (3) angepasst ist, um das distale Ende des Nahtkerns (1) gegen eine Gewebewand zusammenzulegen und zu ankern.

2. Die Nahtvorrichtung (28) des Anspruchs 1, wobei die Nahtkräuselung (2) auf dem Nahtkern (1) mechanisch gekräuselt ist.

3. Eine Nahtanordnung umfassend:
die Nahtvorrichtung (28) nach Anspruch 1 oder 2; und
eine röhrenförmige Nadel (24) umfassend ein inneres Nadellumen, das angepasst ist, um den Nahtkern (1) zu empfangen;
wobei die Nahtkräuselung (2) angepasst ist und Abmessungen hat, um an ein proximales Ende der Nadel anzugrenzen.

4. Die Nahtanordnung des Anspruchs 3, wobei die röhrenförmige Nadel (24) eine vordefinierte Form hat.

5. Die Nahtanordnung des Anspruchs 4, wobei die röhrenförmige Nadel (24) aus einer Formgedächtnislegierung besteht.

6. Die Nahtanordnung des Anspruchs 4, wobei die röhrenförmige Nadel (24) aus Nitinol besteht.

7. Die Nahtanordnung des Anspruchs 4, wobei die röhrenförmige Nadel (24) aus einem superelastischen Material besteht.

8. Ein anastomotisches System umfassend:
eine Nahtvorrichtung nach Anspruch 1; und eine röhrenförmige Nadel umfassend ein inneres Nadellumen zum Empfang des Nahtkerns; und
ein Abgabewerkzeug zur Bereitstellung der röhrenförmigen Nadel (24).

9. Das System des Anspruchs 8, wobei die röhrenförmige Nadel eine vordefinierte Form hat.

10. Das System des Anspruchs 8, wobei die röhrenförmige Nadel eine vordefinierte bogenförmige Gestalt hat.

11. Das System des Anspruchs 9 oder 10, wobei die röhrenförmige Nadel aus einer Formgedächtnislegierung besteht.

12. Das System des Anspruchs 9 oder 10, wobei die röhrenförmige Nadel aus Nitinol besteht.

13. Das System von einem der Ansprüche 8 bis 12, weiterhin umfassend eine Vielzahl von röhrenförmigen Nadeln und eine Vielzahl von Nahtvorrichtungen, wobei jede Nahtvorrichtung in einem inneren Nadellumen einer jeweiligen Nadel empfangen wird.

## Revendications

1. Un dispositif de suture (28), comprenant :
un noyau de suture (1) ayant une lumière intérieure de noyau (101) ;
un bord replié de suture (2) situé dans une extrémité proximale du noyau de suture, le bord replié de suture (2) étant fixé à une suture (102) dans la lumière intérieure de noyau (101) ; et
une gaine d'ancrage (3) située dans une extrémité distale du noyau de suture (1), dans lequel la gaine d'ancrage (3) est compressible dans une direction axiale, est fixée à une extrémité située dans la position la plus distale du noyau de suture (1) et s'étend dans une direction proximale, et dans lequel la gaine d'ancrage (3) est adaptée pour replier et ancrer l'extrémité distale du noyau de suture (1) sur une paroi de tissu.

2. Le dispositif de suture (28) de la revendication 1, dans lequel le bord replié de suture (2) est replié mécaniquement sur le noyau de suture (1).

3. Un ensemble de suture comprenant :
le dispositif de suture (28) selon la revendication 1 ou 2 ; et
une aiguille tubulaire (24) incluant une lumière intérieure d'aiguille adaptée pour recevoir le noyau de suture (1) ;
dans lequel le bord replié de suture (2) est adapté et dimensionné pour jouxter une extrémité proximale de l'aiguille.

4. L'ensemble de suture de la revendication 3, dans lequel l'aiguille tubulaire (24) a une forme prédéfinie.

5. L'ensemble de suture de la revendication 4, dans lequel l'aiguille tubulaire (24) est faite en un alliage à mémoire de forme.

6. L'ensemble de suture de la revendication 4, dans lequel l'aiguille tubulaire (24) est faite en nitinol.

7. L'ensemble de suture de la revendication 4, dans lequel l'aiguille tubulaire (24) est faite en un matériau superélastique.

8. Un système anastomotique, comprenant :
un dispositif de suture selon la revendication 1 ; et une aiguille tubulaire incluant une lumière intérieure d'aiguille pour recevoir le noyau de suture ; et
un outil de placement pour le déploiement de l'aiguille tubulaire (24).

9. Le système de la revendication 8, dans lequel l'aiguille tubulaire a une forme prédéfinie.

10. Le système de la revendication 8, dans lequel l'aiguille tubulaire a une forme arquée prédéfinie.

11. Le système de la revendication 9 ou 10, dans lequel l'aiguille tubulaire est faite en un alliage à mémoire de forme.

12. Le système de la revendication 9 ou 10, dans lequel l'aiguille tubulaire est faite en nitinol.

13. Le système de l'une quelconque des revendications 8 à 12, comprenant en outre une pluralité d'aiguilles tubulaires et une pluralité de dispositifs de suture, chaque dispositif de suture étant reçu dans une lumière intérieure d'aiguille d'une aiguille correspondante.
